# EUROPEAN PATENT APPLICATION

(11) **EP 4 797 274 A1**
(43) Date of publication of application: **26.08.2026**
(21) Application number: 25315064.3
(22) Date of filing: 25.02.2025
(51) Int. Cl.: G16H 20/40, G16H 30/40, A61B 6/12, A61B 6/00, A61B 34/00, A61B 34/30, A61B 90/00

(54) **A COMPUTER-IMPLEMENTED METHOD, A SYSTEM, A COMPUTER PROGRAM PRODUCT, AND A COMPUTER-READABLE STORAGE MEDIUM FOR ASSISTING IN A CARDIAC SURGICAL PROCEDURE**

(71) Applicant: Caranx Medical, 75013 Paris (FR)
(72) Inventor: BERTHET-RAYNE, Pierre, 06800 Cagnes-sur-Mer (FR); IONITA, Silvia, 06700 Saint Laurent de Var (FR); DURAN, Washington Aguirre, 06000 Nice (FR); BLORVILLE, Célia, 06600 Antibes (FR); MUKHINA, Ekaterina, 06000 Nice (FR); BECHAR, Ikhlef, 06410 Biot (FR); MOSCU, Mircea, 06600 Antibes (FR); WEI, Wen, 06410 Biot (FR); HILDENBRAND, Xavier, 06000 Nice (FR)
(74) Representative: Hepp Wenger Ryffel AG

(57) **Abstract**

The invention relates to a computer-implemented method for assisting in a cardiac surgical procedure. The method comprises receiving a plurality of imaging data (2) from an imaging device (21), in particular fluoroscopy imaging data from a fluoroscopy imaging device. The method further includes carrying out at least one pre-validation check. The pre-validation check may comprise automatically determining if a cardiac implant (3), in particular a heart valve implant, is included in the imaging data (2). The pre-validation check may comprise automatically determining if a contrast agent in the cardiac region is detected in the imaging data (2). The pre-validation check may comprise automatically determining if a cardiac anatomic feature (4), in particular the non-coronary cusp (N), is included in the imaging data (2) or can be determined based on the imaging data (2). The method comprises providing an output indicative of at least one result (5) of the at least one pre-validation check, in particular of every pre-validation check, preferably a visual output to be displayed on a display (6).

## Description

The present invention relates to a computer-implemented method, a system, a computer program product, and a computer-readable storage medium for assisting in a cardiac surgical procedure for assisting in a cardiac surgical procedure based on data from an imaging device according to the independent claims. Further embodiments are defined by the dependent claims.

Cardiac surgical implantation procedures, in particular transcatheter aortic valve implantations/replacement (TAVI/TAVR) often necessitates careful assessment of imaging data by a clinician. The imaging data need to be sufficient to provide information about a cardiac implant, in particular a heart valve implant, and a cardiac anatomy of the patient such that the cardiac implant can be reliably navigated through the native anatomy during the cardiac surgical procedure.

However, clinicians performing these steps require highly trained decision making and multitasking skills for successfully navigating and positioning the cardiac implant with respect to the native anatomy. Thus, clinicians require specialized training to acquire expertise to effectively carry out such implantation procedures. Clinicians must simultaneously operate and navigate surgical instruments reliably and efficiently based on an assessment of the complex imaging data while also coordinating and communicating effectively with a team of clinicians. The clinician must engage in simultaneous cognitive and procedural tasks while at the same time necessitating concurrent interpretation of imaging data. This inherently increases a risk of errors or variability in outcomes due to cognitive overload, divided attention of clinicians, and/or misinterpretation of imaging data.

WO 2024/121677 A1 discloses feeding the at least one fluoroscopic image into a machine leaning (ML) model, obtaining an indication of the annulus line of the heart of the subject, and presenting the indication as an overlay of the annulus line over the at least one fluoroscopic image.

However, the prior art lacks a precise computer implemented method which enables a simple, reliable, and efficient assistance in assessing an aptitude of imaging data for a cardiac surgical implantation procedure. Furthermore, the prior art frequently lacks seamless integration with modern robotic controllers, imaging devices, and/or real-time data analytics, resulting in workflow inefficiencies and reduced overall effectiveness.

It is an object of the present invention to overcome these and other disadvantages of the prior art. The present invention shall provide a computer implemented method, system, computer program product, and computer-readable storage medium for assisting in a cardiac surgical procedure which solve the above-mentioned technical problems and enable reliable and simple assessment of the imaging data and rendering of the findings/results.

In particular, it is an object of the present invention to mitigate these risks and increasing a clinician's ability to multitask and reliably to effectively share relevant operational information on the imaging data. Moreover, the invention shall provide enhanced visualization and real-time feedback for clinicians, improve patient safety and outcomes, increase efficiency of the cardiac implantation procedure, enable a broader accessibility reducing dependence on a steep learning curve of clinicians, and enable seamless implementation in typical workflows and systems. In particular, the present invention shall provide relevant spatial information on intra-operative imaging data indicative of a cardiac implant and/or a native anatomy together, preferably with respect to each other, which facilitates performing the cardiac surgical procedure for the clinician and/or a robotic controller.

The computer-implemented method according to the invention comprises receiving a plurality of imaging data from an imaging device, in particular in particular fluoroscopy imaging data from a fluoroscopy imaging device. In a first pre-validation check it is optionally verified if the imaging data has a correct format, dimension, metadata attribute, and/or size. The method further includes, in particular if the imaging data was verified, at least one of the following pre-validation checks. A pre-validation check includes automatically determining if a cardiac implant, in particular a heart valve implant, is included in the imaging data. A pre-validation check includes automatically determining if contrast agent in the cardiac region of the patient is detected in the imaging data. A pre-validation check comprises automatically determining if a cardiac anatomic feature, in particular the non-coronary cusp, is included in the imaging data or can be determined based on the imaging data. The method includes providing an output, preferably a visual output to be displayed on a display, indicative of results of the at least one pre-validation check, in particular of every pre-validation check.

This provides an intuitive interface design and enables automation of repetitive tasks which are difficult to address in parallel to minimize a cognitive load on clinicians.

Verifying the imaging data in a pre-validation check may ensure that minimum requirements for processing the imaging data are fulfilled by the imaging data.

The output indicative of the result may be in the form of a confirmation that the respective pre-validation check has been fulfilled or not, in particular a graphical confirmation such as a check mark/cross or red light/green light.

The output of the result / result(s) of the at least one pre-validation check/check(s) may all be provided sequentially or simultaneously in real-time.

The cardiac anatomic feature comprising the non-coronary cusp, in particular a tip of the non-coronary cusp, may improve the output of the method because contrast agent is often administered in or in proximity of the non-coronary cusp without risk of obstructing the coronary ostia.

If the cardiac implant and/or the cardiac anatomic feature is included in the imaging data or can be determined based on the imaging data, the pre-validation check may further include verifying if the imaging data is sufficient to determine a position, a shape, and/or an orientation of the cardiac implant and/or the cardiac anatomic feature, in particular with respect to each other.

This classification may enable carrying out a simple prior pre-validation check to determine if the imaging data is sufficient for retrieving geometric information of the cardiac implant/cardiac anatomic feature before demanding more computational power and time to actually determine the geometric information.

The position, the shape, and/or the orientation of the cardiac anatomic feature may be determined based on the imaging data indirectly. In particular, imaging data which were not acquired during administration of contrast agent may be used for determining the position, the shape, and/or the orientation based on using a machine learning model by a learned relationship between features visible in this imaging data such as calcifications or a medical instrument such as a pigtail which may be placed in the non-coronary cusp.

The pre-validation check may comprise, in particular if the imaging data were verified to be sufficient, determining a position, shape, and/or orientation of the cardiac implant and/or the cardiac anatomic feature. The pre-validation check may further comprise providing visual data comprising the position, shape, and/or orientation of the cardiac implant and/or the cardiac anatomic feature, in particular with respect to each other.

This determination of the position(s),' the shape(s), and/or the orientation(s) enables a clinician to avoid time-consuming and error-prone identification of the cardiac implant/cardiac anatomic feature. The cardiac anatomic feature may further be determined more reliably based on determining if contrast agent is detected in the cardiac region in the imaging data before determining the position, the shape, and/or the orientation of the cardiac anatomic feature.

This determination of the position(s), the shape(s), and/or the orientation(s) may be partially or fully automatic.

The pre-validation check may comprise receiving a user input indicative of a longitudinal location on the cardiac implant, in particular in the form of a percentage of a total longitudinal length of the cardiac implant. The pre-validation check optionally comprises receiving another user input indicative of a type and/or a size of the cardiac implant. The pre-validation check may further include processing the position, the shape, and/or the orientation of the cardiac implant to determine a geometric cardiac implant characteristic which is indicative of the longitudinal location. The pre-validation check may further include generating and providing visual data including data for displaying the geometric cardiac implant characteristics in the imaging data as a graphical representation. The graphical representation preferably has a width perpendicular to a longitudinal axis of the cardiac implant at a reference point of the cardiac implant in the imaging data corresponding to the longitudinal location of the user input. The graphical representation may optionally have a length of the cardiac implant parallel to the longitudinal axis of the cardiac implant. The width, and in particular the length, optionally correspond to the cardiac implant in a fully expanded configuration and are determined based on the other user input of the type and/or the size of the cardiac implant.

The user input may be indicative of a variable longitudinal location on the cardiac implant,e.g. a percentage of a total current longitudinal length of the cardiac implant, such that the variable longitudinal location is continuously adjusted according to the current longitudinal length of the cardiac implant in the imaging data in real-time. This may provide the clinician with a visual indicator which takes non-uniform expansion and longitudinal/lateral displacement of the cardiac implant during movement of the cardiac implant to a fully expanded configuration into account.

The method may comprise providing visual data indicative of the type and/or the size based on the other user input and/or the longitudinal location on the cardiac implant based on the user input and preferably displaying this visual data.

Alternatively, to the shape and/or the size of the cardiac implant being provided based on the other user input, the method may comprise automatically determining the type and/or the size of the cardiac implant based on the determined position, the shape, and/or the orientation of the cardiac implant in the imaging data.

This visual data including the graphical representation determined based on the user input, and in particular the other user input, may enable the clinician to manually select a longitudinal location on the cardiac implant. Thereby, a clinician can reliably be provided with visual data comprising the graphical representation of the cardiac implant which has been customized based on the user input(s) such that a clinician's expertise, the type/size of the cardiac implant, and/or a patient-specific indication can be taken into consideration. Thereby, the clinician can adjust a target implantation depth accordingly to the specific needs of the patient. This renders the method more versatile and customizable. This further enables to retrieve spatial information of the input longitudinal location on the cardiac implant with respect to the cardiac anatomic feature reliably from the displayed visual data as real-time feedback.

The visual output may comprise at least one graphical representation of the cardiac implant characteristic of the heart valve implant which is configured to be overlaid with the imaging data and/or at least one position/orientation of the cardiac anatomic feature. This visual output may be configured to be displayed next to or adjacent the imaging data in a user interface.

The pre-validation check may optionally comprise receiving a user input indicative of a type and/or size of the cardiac implant. The pre-validation check may comprise generating a predicted deployed configuration of the cardiac implant in the fully expanded configuration based on the position, the shape, and/or the orientation of the cardiac implant with respect to the imaging data. The predicted deployed configuration optionally comprises or consists of a computer visualization of the cardiac implant in the fully expanded configuration. The pre-validation check may optionally comprise compensating a position of the predicted deployed configuration of the cardiac implant for a longitudinal displacement with respect to the current position of the cardiac implant in the imaging data caused by non-uniform expansion pattern of the cardiac implant, in particular based on the type and/or size of the cardiac implant. The pre-validation check may comprise providing visual data comprising the predicted deployed configuration, in particular compensated for the non-uniform expansion pattern.

This may enable clinicians to easily and reliably determine a predicted deployed configuration of the cardiac implant in the fully expanded configuration based on imaging data displaying the cardiac implant in a non-deployed or merely partially de-played configuration. Thereby, the position/orientation of the cardiac implant may be adjusted and optimized prior to expanding the cardiac implant.

Reference is made to the European patent application No. EP 24315363.2 filed on 30 Juli 2024 which discloses predicting an intra-operative future deployment state, denoted as predicted deployment configuration in this application, based on a non-uniform expansion pattern. In particular, reference is made to determining the intra-operative future deployment state by taking into consideration the movement of a geometric center of the cardiac implant and/or a change in orientation of the cardiac implant.

Compensating for the non-uniform expansion pattern of the cardiac implant further enables clinicians to easily take into consideration displacement due to inherent non-uniform expansion patterns of the different cardiac implants, e.g. due to different types/sizes of cardiac implants such as heart valve implants by different manufacturers. The cardiac implant may have a structural design which results in certain non-uniform expansion patterns, e.g. different longitudinal locations of the cardiac implant radially expanding to a different degree when expanding the cardiac implant. This may result in a different relative longitudinal shortening of different longitudinal sections of the cardiac implant which is difficult to predict and compensate merely based on a clinician's assessment.

In addition or alternative, the position of the predicted deployed configuration of the cardiac implant may be compensated in the pre-validation check for lateral displacement with respect to the current position of the cardiac implant in the imaging data caused by the non-uniform expansion pattern of the cardiac implant, in particular based on the type and/or size of the cardiac implant. The lateral displacement may occur due to the interaction of the cardiac implant, e.g. the heart valve implant, with the cardiac anatomic feature due to displacement relative to a native cardiac body duct. For example, the lateral displacement may be due to a collapsed heart valve implant being placed in an eccentric configuration with respect to the native cardiac body duct prior to deployment such that expanding the heart valve implant moves the geometric center of the heart valve implant towards a more concentric configuration.

The method may comprise prompting the user to determine if the user wants to display the predicted deployed configuration of the cardiac implant or not and displaying the predicted deployed configuration accordingly.

The method may further comprise prompting the user to determine if the user wants to display the predicted deployed configuration of the cardiac implant which was compensated for the longitudinal/lateral displacement or not.

The pre-validation check may comprise generating a landing zone of the cardiac implant in a fully expanded configuration based on the position, the shape, and/or the orientation of the cardiac anatomic feature with respect to the imaging data. The landing zone, in particular comprises or consists of a computer visualization of the cardiac implant. The pre-validation check may optionally comprise receiving a user input indicative of a longitudinal location on the cardiac implant, in particular in the form of a percentage of a total longitudinal length of the cardiac implant. The pre-validation check may optionally comprise compensating the landing zone of the cardiac implant based on the longitudinal location on the cardiac implant. The pre-validation check may include providing visual data comprising the landing zone.

This may enable the clinician to reliably identify a target zone in the displayed visual data without necessitating prior experience, specialized training, or a learning curve. Displaying the visual data comprising the landing zone may further facilitate carrying out the surgical procedure. It may be synergistic and beneficial if visual data including the predicted deployed configuration and the landing zone are displayed together, in particular in real-time, preferably as a similar but discernible computed visualization, such that these can be aligned prior to expanding the cardiac implant.

The method may comprise prompting the user to determine if the user wants to display the landing zone of the cardiac implant or not and displaying the landing zone accordingly based on this user input.

The method may comprise providing visual data comprising a warning, if a position of the predicted deployed configuration deviates more than a pre-defined amount, in particular more than 3 mm, preferably more than 2 mm, from a position of the landing zone, in particular in a longitudinal direction of the cardiac implant.

The computed visualization of the predicted deployed configuration or the target landing zone may be a rectangular model and/or a high-fidelity model.

The method may comprise determining a distance between (i) the position of the cardiac implant or the previously described geometric cardiac implant characteristic and (ii) the position of the cardiac anatomic feature in a projection on the longitudinal axis. The visual data comprising the longitudinal projected distance may be provided.

This longitudinal projected distance may enable an eccentrically arranged cardiac anatomic feature, such as a cusp of a native heart valve, in particular the non-coronary cusp, to be used for determining a metric indicative of a distance of the cardiac implant a target location, e.g. the aortic heart valve.

The method may comprise determining an eccentricity between the position of the cardiac implant or the previously described geometric cardiac implant characteristic and the position of the cardiac anatomic feature or another cardiac anatomic feature. The visual data comprising the eccentricity may be provided.

The cardiac anatomic feature, in particular for determining the eccentricity, may comprise or consist of the aortic heart valve or parts thereof, in particular the cusp(s), the mitral heart valve, the tricuspid heart valve, the pulmonary heart valve, the coronary ostia, the aortic root, the ascending aorta, the sinotubular junction, and/or the left ventricular outflow tract.

This visual data including the eccentricity may provide the clinician with a simple visualization of eccentric displacement of the cardiac implant within a cardiac anatomic feature. This may enable clinicians to remedy this eccentric displacement, i.e. arrange the cardiac implant more concentrically with respect to the cardiac anatomic feature, such as the aortic heart valve, prior to expansion of the cardiac implant. Thereby, the cardiac implant may be expanded with less interaction with the anatomy, leading to reduced displacement and a more reliable deployment position/orientation of the cardiac implant and a reduced risk of plaque dislodgement. This may e.g. be achieved due to commercially available heart valve implants such as Edwards "SAPIEN 3" enabling tilt adjustments to position the heart valve implant with respect to the valve. The method may comprise sending control commands to a robotic controller to adjust the tilt accordingly to arrange the cardiac implant more concentrically with respect to the cardiac anatomic feature.

A user may provide a user input of the distance metric, e.g. meter or percent of the cardiac implant length, which is used to display the longitudinal projected distance or the eccentricity.

The pre-validation check may comprise determining if a pigtail is positioned within one of the cusps, preferably determine if the pigtail is positioned within the non-coronary cusp, the right coronary cusp, or the left coronary cusp.

This may effectively reduce the workload for clinicians and allow clinicians to focus on higher-level decision-making. The result indicating that the pigtail is positioned within one of the cusps may also enable a more exact determination of the position, the shape, and/or orientation of the cardiac anatomic feature by this pigtail providing a consistent anatomic reference, in particular irrespective of a cardiac or respiratory cycle of the patient.

The pre-validation check may comprise processing of the imaging data to determine if the cardiac implant or the geometric cardiac implant characteristic is arranged in proximity of (i) the cardiac anatomic feature and/or (ii) the previously described pigtail. The pre-validation check may determine if the cardiac implant or the geometric cardiac implant characteristic is arranged spaced apart less than 5 cm, in particular less than 3.5 cm, preferably less than 2 cm, from the cardiac anatomic feature.

This may enable automated notification of this result to the clinician to indicate that the cardiac implant is in a region of interest for implantation. The distance these are spaced apart at may be the longitudinal projected distance.

The method may comprise automatically carrying out at least one pre-validation check, in particular a plurality of the previously described pre-validation checks, based on a positive result indicating that the cardiac implant is in proximity of the cardiac anatomic feature/pigtail. Thereby, the method can automatically assist the clinician(s) once this region of interest for implantation is reached.

The method may comprise providing a user notification that a manual, partially automated, or fully automatic operation of the robotic controller may be used if the cardiac implant is arranged in the proximity of the cardiac anatomic feature and/or the pigtail.

The method may comprise prompting a user to select a non-automatic, partially automatic, or fully automated operation of the robotic controller if the cardiac implant is arranged in the proximity of the cardiac anatomic feature and/or the pigtail.

The pre-validation check may comprise processing the imaging data to determine if the imaging data were acquired in a two-cusp view, in a three-cusp view, or in another view. The method optionally comprises generating at least one control instruction to move the imaging device to achieve the two-cusp view or the three-cusp view. The output may comprise the at least one control instruction. In addition or alternative, at least one control command for the imaging device may be generated and transmitted to the imaging device to achieve the at least one control instruction. The method may optionally comprise providing visual data to be displayed on a display indicative of whether the imaging data were acquired in the two-cusp view, in the three-cusp view, or in another view.

In the two-cusp view, an imaging perspective of the imaging device is aligned with respect to the patient in such a manner that two of the aortic valve cusps, typically the left and right coronary cusp, appear co-planar and overlapping in two-dimensional imaging data. In the three-cusp view, the imaging perspective is aligned such that all three aortic valve cusps are distinctly visible and separated from each other, typically equidistantly separated from each other.

Providing the output comprising result of this pre-validation check indicative of in which view the imaging data were acquired may facilitate verification and assessment of the relevant views for cardiac surgical procedures. Displaying the output comprising the control instructions to the clinician or generating at least one control command to achieve the control instructions may facilitate and expedite adjusting the imaging device to achieve the respective view.

The computer-implemented method may comprise receiving a user input indicative of a type of the imaging device and/or parameters indicative of a characteristic of the imaging device, in particular a detector size, a source-to-image distance, a source-to-object distance, an angular positioning, a collimation, and/or a magnification factor. The method may comprise providing visual data for a user of the type of imaging device and/or parameters indicative of the characteristic.

The pre-validation check may comprise receiving or retrieving status data from at least one robotic controller. The status data may be indicative of an actuation direction of the at least one medical instrument driven by at least one actuator of the robotic controller. In addition or alternative, the status data may be indicative of sensor data indicative of slippage of the at least one actuator driving the at least one medical instrument. In addition or alternative, the status data may be indicative of a status of the robotic controller, in particular an operational status indicating if the robotic controller is operated manually, partially automatically, or fully automatically. In addition or alternative, the status data may be indicative of an inflation status of a balloon catheter for deploying the cardiac implant, in particular including a volume of inflation fluid used for inflation. In addition or alternative, the status data may be indicative of a contrast agent status, in particular including a volume of contrast agent administered. In addition or alternative, the status data may be indicative of a torque of the at least one actuator for driving the at least one medical instrument. The status data is in particular processed for determining a prediction position, shape and/or orientation of the cardiac implant or the geometric cardiac implant characteristic based on the status data and providing visual data comprising the predicted position, shape, and/or orientation.

This may simplify system integration and may enhance efficiency of clinicians by including the functionalities/information of a robotic controller in form of status data in the output/visual data of this computer-implemented method.

The status data and the position, the shape, and/or the orientation of the cardiac implant in the imaging data may both be used in a closed feedback loop to determine a more accurate position, shape, and/or orientation of the cardiac implant in the imaging data.

The method may comprise determining a body duct outline, in particular an aorta outline, based on the imaging data, optionally using a pre-trained machine learning model. The body duct outline is determined based on at least one of (i) prior and/or current imaging data acquired during administration of contrast agent, (ii) prior and/or current imaging data acquired without administration of contrast agent, and (iii) pre-operational imaging data preferably computer tomography imaging data. The visual data comprising the body duct outline are provided to be overlaid on the imaging data.

This may allow for a simple and reliable identification of the body duct(s) relevant to the cardiac surgical procedure without placing an undue burden for clinicians.

The visual data comprising the body duct outline determined based on the imaging data acquired during administration of contrast agent may in particular be overlaid on the imaging data which was acquired without administration of contrast agent, e.g. after the contrast agent dissipated. Such an "after-image-overlay" may enable a more effective consideration of the body duct outline throughout the cardiac surgical procedure irrespective of further contrast agent administration.

The determination of the body duct based on imaging data without administration of contrast agent may be achieved by a machine learning model which was trained using test imaging data without contrast agent and validated using validation image data comprising the body duct outline for a large number of patients.

The pre-operational imaging data may be three-dimensional pre-operational computer tomography data which are registered to the two-dimensional fluoroscopy imaging data received in the surgical procedure. This may enable taking into consideration a spatial position of cardiac and/or vascular anatomical features not sufficiently visible in two-dimensional image data, such as a spatial position, shape, and/or orientation of the coronary cusps, in particular the non-coronary cusp, the coronary septum and/or the coronary ostia.

The method may comprise determining and providing visual data for displaying parameters of the vasculature, in particular the aorta and/or the aortic valve. The parameters of the vasculature may be determined based on the imaging data, in particular the imaging data during administration of contrast agent or the imaging data without administration of contrast agent and a machine learning model trained to predict the parameters of the vasculature based on non-contrast imaging data. The parameters of the vasculature may comprise at least one diameter of the aortic valve annulus, the sinuses of Valsalva, the sinotubular junction, the ascending aorta, the left ventricular outflow tract, and/or the aortic arch.

The method may comprise visually displaying a prompt for providing a user input to indicate if the method is to be carried out with or without contrast agent. Different machine learning models may be applied for carrying out the method depending on the user input.

The method may comprise determining at least one position and/or shape of a calcification and providing visual data indicative of the position and/or shape of the calcification. This visual data may be overlaid over the imaging data.

The computer implemented method may comprise providing visual data to be displayed comprising an input interface which enables a user to create, modify delete and/or organize note entries. The method may also comprise providing visual data to be displayed comprising specific patient information such as the name, age, indication, comorbidities, and/or previous surgeries.

The pre-validation check may comprise determining if rapid pacing is successfully applied to the patient, in particular based on (i) the imaging data, (ii) heartbeat sensor data, and/or (iii) received rapid pacing control data for operating a rapid pacing device.

This may enable to ensure that rapid pacing is correctly applied before crossing a heart valve with the cardiac implant such that adverse patient outcomes, e.g. due to detachment of plaque, are minimized.

The heartbeat provided by the heartbeat sensor data of the patient may be provided as visual data to be displayed on the display. The visual data for displaying data indicative of the cardiac implant position and/or the anatomic cardiac feature position may be configured for increasing and decreasing in brightness/changing colour in accordance with the heartbeat.

The method may comprise providing the imaging data to be displayed on a display, in particular together with the visual output. The imaging data may be overlaid with the previously described at least one position, shape, and/or orientation of the cardiac implant and/or the cardiac anatomic feature. In addition or alternative, the imaging data may be overlaid with the previously described geometric cardiac implant characteristic. In addition or alternative, the imaging data may be overlaid with the previously described predicted deployed configuration. In addition or alternative, the imaging data may be overlaid with the previously described generated landing zone. In addition or alternative, the imaging data may be overlaid with a visual indicator indicative of the previously described longitudinal projected distance or the previously described eccentricity. In addition or alternative, the imaging data may be overlaid with the previously described position, shape, and/or orientation of the cardiac implant and/or the geometric cardiac implant characteristic predicted based on the status data. In addition or alternative, the imaging data may be overlaid with the previously described body duct outline.

Displaying this imaging data together with at least one of the previously described overlays may increase procedural efficiency and streamline the cardiac surgical procedure. Displaying these may further reduce the risk of cognitive overload by displaying relevant data together in a common graphical user interface while keeping secondary metrics in the imaging data still accessible for the clinician.

This visual data to be overlaid onto the imaging data may be provided and displayed in real-time.

Providing this imaging data and the previously described visual data to be overlaid together with the output(s) may further reduce complexity and enable displaying imaging data and procedural results in the common graphical user interface.

The method may comprise (i) generating visual data comprising a warning or (ii) generating a control command for preventing actuation of the robotic controller and transmitting the control command to the robotic controller if the predicted position, shape, and/or orientation of the cardiac implant or the geometric cardiac implant characteristic is spaced apart beyond a predetermined threshold distance relative to the cardiac anatomic feature. The predetermined threshold distance may be less than 3 mm, in particular less than 2 mm, preferably less than 1 mm.

This may ensure that the cardiac implant is not malpositioned with respect to the cardiac anatomic feature and/or the clinician is at least notified of a risk of malpositioning of the cardiac implant.

Alternatively, the method may comprise (i) generating visual data comprising a warning or (ii) generating a control command for preventing actuation of the robotic controller and transmitting the control command to the robotic controller if the predicted deployed configuration of the cardiac implant is spaced apart beyond a predetermined threshold distance relative to the landing zone. The predetermined threshold distance may be less than 3 mm, in particular less than 2 mm, preferably less than 1 mm.

The previously described threshold distance may be the longitudinal projected distance.

The method may comprise determining a confidence status based on the at least one result of the at least one pre-validation check to classify a suitability of the imaging data or the at least one result for performing a placement of the cardiac implant in a heart valve, in particular the heart valve implant in the aortic heart valve. At least three confidence levels indicating at least three different suitability regimes can be provided. Visual data indicative of the confidence levels may in particular be provided. In particular, visual data indicative of the confidence levels for displaying a bar diagram and/or a colour scheme, in particular a traffic light colour scheme is provided.

This may provide clinicians with a simple and robust metric to determine a reliability of the output/visual data provided by the computer-implemented method.

The method may comprise determining a contrast agent confidence status indicative of an amount of contrast agent in the cardiac region, in particular in or adjacent of the heart valve, preferably in or adjacent the aortic heart valve, in the imaging data. The method may comprise providing at least three contrast confidence levels indicating at least three different regimes of contrast agent amounts. The visual data indicative of the contrast confidence levels may be provided. In particular, visual data for displaying a bar diagram and/or a colour scheme, in particular a traffic light colour scheme, indicative of the contrast confidence levels may be provided.

This may simplify assessment whether the vasculature is sufficiently visible based on contrast agent administration for performing the surgical procedure.

The confidence level and/or the contrast confidence level may be determined and preferably displayed in real-time.

The method may comprise determining an amount indicative of (i) a total X-ray exposure and/or (ii) contrast agent administration based on the imaging data and providing visual data comprising this amount to be displayed on the display.

This may enable a clinician to track and avoid exceeding this amount, e.g. to avoid excess administration of contrast agent which may lead to contrast-induced nephropathy.

The method may comprise processing the imaging data such that the cardiac implant included in the imaging data is displayed in a center of a displayed image based on the imaging data and providing the centered imaging data to be displayed on the display.

This may provide clinicians with a motion stabilized view of the cardiac implant with respect to the anatomy of the patient, ensuring that the centered imaging data includes a clear and easily discernible display of the cardiac implant.

Another aspect of the invention relates to a computer-implemented method which facilitates displaying only the relevant imaging data throughout the cardiac surgical intervention, in particular a heart valve implantation/replacement, preferably an aortic heart valve implantation/replacement.

The computer-implemented method, in particular a previously described method, may comprise determining a distance between a current position of the cardiac implant and a current position of the cardiac anatomic feature. The imaging data is pre-processed to be displayed by adjusting a zoom level before providing it such that the zoom level is higher when the distance is smaller and lower when the distance is larger. The zoom level is preferably chosen such that the cardiac implant and the cardiac anatomic feature are included or can be determined based on the pre-processed imaging data irrespective of the zoom level.

This can facilitate a more comprehensive display of imaging data in the initial stages of the surgical procedure when advancing medical instruments through the vasculature, e.g. transfemorally through the descending aorta, while enabling a more precise, localized assessment of the imaging data in the subsequent steps, e.g. deployment of the heart valve implant in the aortic heart valve.

The method may comprise continuously detecting a trajectory of the position of the cardiac implant with respect to the imaging data and processing the detected trajectories to determine a past trajectory. Alternatively or additionally, the method may comprise predicting a future trajectory of the cardiac implant with respect to the imaging data based on the position of the cardiac anatomic feature, and in particular the body duct outline. The method may comprise providing visual data for displaying the past/future trajectory.

The visual data for displaying the future and the past trajectory may be displayed using different colours to enable clear differentiation between them. Continuously detecting the trajectory of the cardiac implant may comprise detecting if the trajectory was navigated manually or via the robotic controller. The trajectory navigated manually may be displayed discernible from, in particular in a different colour, than the trajectory navigated by the robotic controller.

This may provide the clinician with enhanced oversight of the surgical procedure and simplify accurate tracking of the medical instrument navigation.

The method may comprise determining a paravalvular leakage metric indicative of paravalvular leakage of a fully deployed cardiac implant after the cardiac implant was converted to the fully expanded configuration based on the imaging data, in particular imaging data acquired under the influence of contrast agent. Visual data for displaying the paravalvular leakage metric may be provided.

Paravalvular leakage during valve implantations, in particular after TAVI, negatively impacts short- and long-term survival. Reliably detecting this paravalvular leakage metric may validate whether the heart valve implant is optimally positioned and/or has the correct type/size corresponding to the native anatomy dimensions.

The method may comprise sequentially providing display data indicative of which pre-validation check is currently carried out, and in particular which pre-validation check was carried out already, preferably together with the corresponding output indicative of the result of the corresponding pre-validation check.

This enables cognitive load reduction for clinicians while providing a step-by step structured visualization of each procedural prerequisite. Moreover, this may bring negative results of one of the pre-validation checks or visual data directly to the attention of the clinician(s) such that these prerequisites may be efficiently rectified. The display data indicative of which pre-validation check is currently carried out may be provided in real time.

The method may comprise highlighting the current pre-validation step which is currently processed.

Instead of visually displaying the results of every pre-validation check sequentially, the method may be configured to only show which pre-validation check is still missing, currently carried out, and/or has a negative result.

The method may comprise determining (i) the inflation status of the balloon catheter for deploying the cardiac implant, in particular the volume of inflation fluid used for inflation, and/or (ii) the contrast agent status, in particular including a volume of contrast agent administered based on the imaging data. The method further comprises providing visual data to be displayed on the display indicative of the inflation status and/or the contrast agent status.

This may provide a simple and reliable approximation of the inflation status/contrast agent status without relying on data from an inflation device for inflating the balloon catheter or an administration device for administering contrast agent.

Another aspect of the invention relates to a system configured for carrying out the previously described computer-implemented method. The system comprises a control unit for receiving the imaging data and providing the output, and in particular the visual data. The system in particular comprises a display for displaying the output, and in particular the visual data. The system preferably further comprises a previously described robotic controller connected to the control unit.

The control unit may be configured for providing visual data of a graphical user interface which includes the previously described imaging data and the results side by side. The imaging data may further be overlaid with one of the previously described overlays.

Another aspect of the present invention relates to a computer program product which, when the program is executed by a computer, causes the computer to carry out the previously described steps of the computer-implemented method.

Another aspect of the present invention relates to a computer-readable storage medium comprising instructions which, when executed by a computer, cause the computer to carry out the previously described steps of the computer-implemented method.

In cardiac surgical procedures it is often necessary to adjust the position, shape, and/or orientation of a cardiac implant with respect to the native anatomy to conform to a target native anatomy of a patient or to comply with a specific indication of the patient. However, the prior art lacks a computer-implemented method which enables patient-specific and customizable assistance for the cardiac surgical implantation procedure in form of reliable visual information indicative of a cardiac implant and the native anatomy.

It is an object of another aspect of the invention to overcome these and other disadvantages of the prior art. This aspect of the present invention relates to a computer-implemented method for providing visual information to a user for assistance in a cardiac surgical procedure. The method comprises receiving a user input indicative of a longitudinal location on a cardiac implant, in particular a percentage of a total longitudinal length of the cardiac implant. The method optionally comprises receiving a user input indicative of a type and/or size of the cardiac implant. The method comprises receiving a plurality of imaging data from an imaging device, in particular a fluoroscopy imaging device. A position, shape, and/or orientation of the cardiac implant is determined based on the imaging data. The position, the shape, and/or the orientation of the cardiac implant is processed based on the user input to determine a geometric cardiac implant characteristic which is indicative of the longitudinal location. Visual data including data for displaying the geometric cardiac implant characteristic in the imaging data as a graphical representation is generated and provided. The graphical representation preferably has (i) a width perpendicular to a longitudinal axis of the cardiac implant at a reference point of the displayed cardiac implant corresponding to the longitudinal location of the user input, and optionally also (ii) a length of the cardiac implant parallel to the longitudinal axis of the cardiac implant. The width, and in particular the length, optionally correspond to the cardiac implant in a fully expanded configuration and may be determined based on the other user input indicative of a type and/or a size of the cardiac implant.

This may enable clinicians to adjust the graphical representation based on their expertise, the selected valve type/size, and patient-specific needs. This graphical representation correspondingly chosen to the input longitudinal location may assist a clinician in achieving a desired implantation depth of the cardiac implant in the patient's cardiac anatomy more reliably.

The method may comprise providing visual data indicative of the type, the size, and/or the user input indicative of the length of the longitudinal subsection and preferably displaying this visual data. The method may further comprise providing visual data comprising the predicted deployed configuration and/or the landing zone.

The method may comprise providing the imaging data to be displayed on a display, in particular together with the visual data, and preferably with the other user input indicative of the type and/or the size of the cardiac implant. The imaging data are preferably overlaid with the data for displaying the geometric cardiac implant characteristic.

This may enable to keep all clinicians of the surgery informed of the most pertinent information and simplify carrying out the cardiac surgical procedure.

The method may comprise receiving a user input indicative of a volume of inflation fluid to be used for inflation of a balloon catheter and/or a volume of contrast agent, in particular a maximum volume, to be administered during the cardiac surgical procedure. The method may optionally comprise receiving status data indicative of the volume of inflation fluid used for inflation of a balloon catheter and/or the volume of contrast agent administered during the cardiac surgical procedure. The visual data indicative of the volume, in particular corrected for the volume used and/or administered, may be provided to be displayed on a display.

This may enable simple and reliable tracking of the volume of inflation fluid/contrast agent used throughout the cardiac surgical procedure such that a clinician can allocate more capacity to other more pertinent procedural steps of the cardiac surgical procedure.

Another aspect of the present invention relates to a computer program product which, when the program is executed by a computer, causes the computer to carry out the steps of the previously described computer-implemented method.

Another aspect of the present invention relates to computer-readable storage medium comprising instructions which, when executed by a computer, cause the computer to carry out the previously described steps of the computer-implemented method.

Alternatively in another aspect according to the invention, the method may not comprise the steps relating to processing the position, shape, and/or orientation of the cardiac implant based on the user input to determine a geometric cardiac implant characteristic which is indicative of the longitudinal location and generating and providing visual data including data for displaying the geometric cardiac implant characteristic in the imaging data as a graphical representation, preferably compensated for the non-uniform expansion pattern.

Instead, the method may comprise the previously described steps of generating a predicted deployed configuration in the fully expanded configuration based on the position, the shape, and/or the orientation of the cardiac implant with respect to the imaging data. In addition or alternative, the method may comprise the previously described steps of generating a landing zone of the cardiac implant in a fully expanded configuration which is compensated based on the user input indicative of the longitudinal location on the cardiac implant.

Further embodiments of the invention and improvement of the described embodiments will become apparent with the following description of the embodiments.

The invention is now described with reference to certain embodiments and figures which show:
Figure 1: a schematic illustration of a system for performing a computer-implemented method according to the invention,
Figure 2A: an embodiment of a graphical user interface for performing the computer-implemented method according to the invention which carries out sequential pre-validation checks based on the imaging data,
Figure 2B: the graphical user interface according to Fig. 2A providing positive results of the pre-validation checks and displaying an overlay on the imaging data which include a cardiac implant being advanced through the ascending aorta,
Figure 2C: the graphical user interface according to Figs. 2A and 2B displaying an overlay on the imaging data including a cardiac implant being advanced across the native aortic heart valve,
Figures 3A - 3G: an embodiment of a prompt of the graphical user interface for performing the computer-implemented method according to the invention for providing user inputs indicative of the type and size of the cardiac implant and indicative of a longitudinal location,
Figures 4A and 4B: another embodiment of a graphical user interface for performing the computer-implemented method according to the invention including a predicted deployed configuration and a landing zone,
Figure 5A: a schematic illustration of a body duct outline overlaid onto the fluoroscopy imaging data acquired in the three-cusp view,
Figures 5B and 5C: schematic illustrations of the three-cusp and two-cusp view,
Figures 6A and 6B: a schematic illustration of centered imaging data being displayed on a display, and
Figures 7A and 7B: a schematic illustration of imaging data which were pre-processed to adjust a zoom level based on a distance between a cardiac implant and a cardiac anatomic feature.

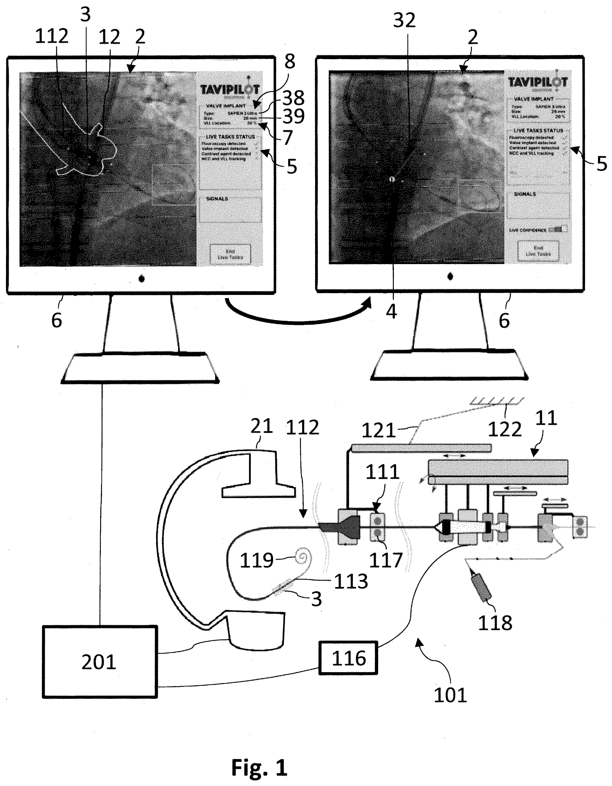

Figure 1 shows a schematic illustration of a system 101 for performing the computer implemented method according to the invention.

The system 101 comprises a control unit 201 for receiving two-dimensional fluoroscopy imaging data 2 from a fluoroscopic imaging device 21 formed by a C-arm. In a first pre-validation check it is verified that the imaging data 2 has the correct format and dimensions corresponding to be processed in the computer-implemented method according to the invention. The imaging data may be pre-processed, in particular using filters and/or cropping the imaging data 2.

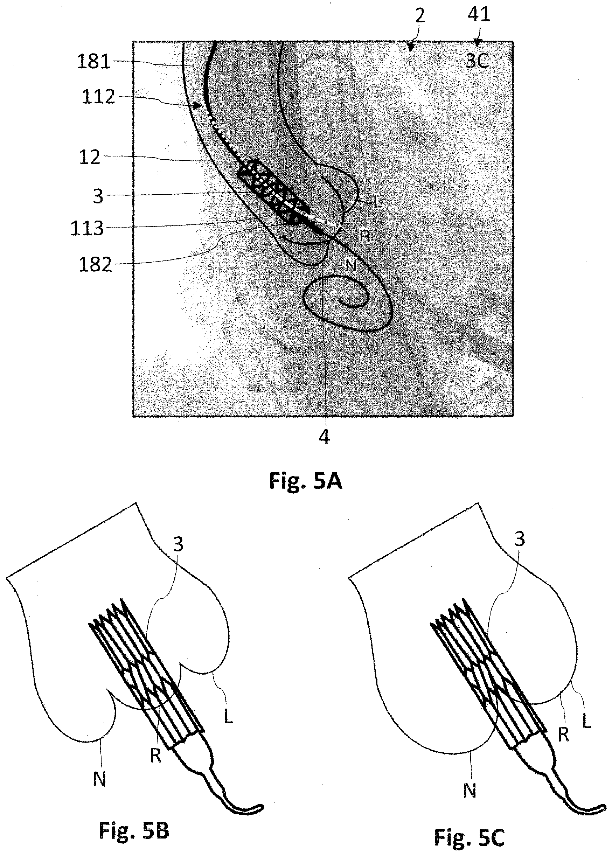

The control unit 201 is further connected to a display 6 for displaying a graphical user interface including the fluoroscopy imaging data 2, results 5 of pre-validation checks, and overlaid data for the fluoroscopy imaging data 2, e.g. a body duct outline 12 such as the aortic hull which includes the coronary ostia and the aortic cusps in a two-cusp view. The imaging data 2 with overlaid visual data and the results 5 are depicted next to each other in the graphical user interface. The control unit 201 is bidirectionally connected to the imaging device 21. The control unit 201 may provide control instructions on to be displayed or transmitted to the imaging device 21 to achieve the two-cusp view or a three-cusp view (see Fig. 5A for further details). The control unit 201 is further connected to a robotic controller 11 for receiving status data 116 from the robotic controller 11. The control unit 201 can further be bidirectionally connected to the robotic controller 11 to transmit control commands in a fully or partially automated mode of operation.

The imaging data 2 in Fig. 1 are acquired during a TAVI/TAVR procedure and, in addition to the native anatomy of the patient, include medical instruments 112 for performing this procedure and an expandable cardiac implant 3 formed by an aortic heart valve implant.

The left side of Fig. 1 shows fluoroscopy imaging data 2 including the cardiac implant 3 in a collapsed configuration being displayed on the display 6. The right side of Fig. 1 shows fluoroscopy imaging data 2 including the cardiac implant 3 in an expanded configuration. The display 6 on the right side displays exemplary imaging data 2 after a successfully completed TAVI/TAVR procedure which is illustrated by the curved black arrow. Once, the TAVI/TAVR procedure is completed, the cardiac implant 3 was converted to a fully expanded configuration in the aortic heart valve.

The graphical user interface displayed on these displays 6 provides visual data indicative of a user input 8 such as relating to a type 38 of a cardiac implant 3, such as "SAPIEN 3 Ultra" from "Edwards", a size 39 of the cardiac implant such as "26 mm", and a user input 7 indicative of a longitudinal location on the cardiac implant 3 such as "20 %". The user input 7 is indicative of a longitudinal location on the cardiac implant corresponding to a relative percentual distance of the current total length of the cardiac implant 3 from a distal end of the cardiac implant 3.

The graphical user interface further provides the results 5 of the pre-validation checks. In particular, Fig. 1 shows the graphical user interface sequentially displaying to the clinician the pre-validation check which is currently carried out and the results of previous pre-validation checks. Figure 1 shows that the pre-validation checks and the results 5 are computed and provided in a predefined order sequentially. First, the control unit 201 of the system 101 verifies that the imaging data 2 are in fact received by the control unit 201. Subsequently, another pre-validation check is carried out to determine if the cardiac implant 3, e.g. an aortic valve implant to be implanted in the native aortic valve, can be reliably detected based on the imaging data 2, in particular by detecting locations of proximal and distal edge markers of the cardiac implant 3. Then, another pre-validation check is performed to determine if contrast agent administration can be detected in the aortic root of the patient based on the imaging data 2.

In a further pre-validation check it is determined whether a cardiac anatomic feature 4 formed by the non-coronary cusp can be detected. The results 5 of these pre-validation checks are provided and displayed as checkmarks for positive results and crosses for negative results. The pre-validation check further includes determining if a geometric cardiac implant characteristic 32 which is denoted as "VLL" in Fig. 1 can be reliably tracked based on the fluoroscopy imaging data 2.

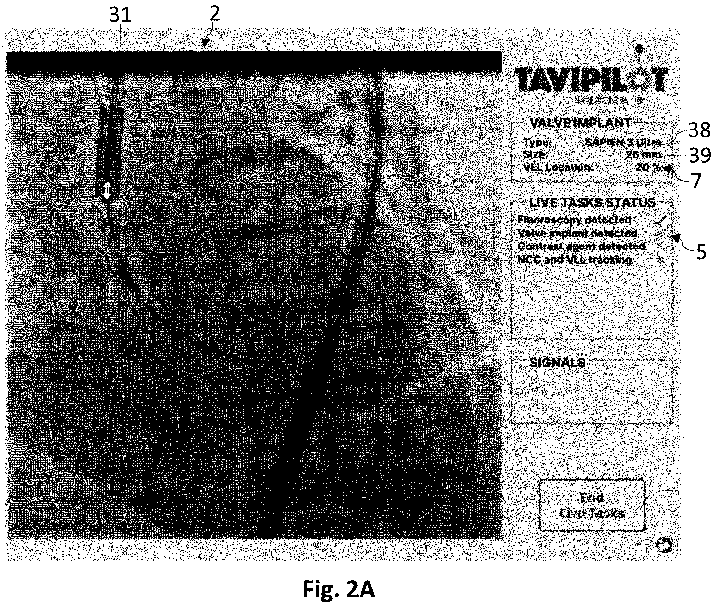

The graphical user interface in the left display 6 exemplary provides a negative result for the pre-validation checks for detecting the cardiac implant 3, the contrast administration, and tracking of the cardiac anatomic feature 4 and the geometric cardiac implant characteristic 32. The graphical user interface in the right display 6 exemplary provides only positive results indicated by checkmarks. Consequently, the cardiac anatomic feature 4 and a representation of the geometric cardiac implant characteristic 32 are only included on the display on the right side of Fig. 1. For enlarged exemplary results based on different imaging data 2 of a similar graphical user interface see Figs. 2A to 2C.

Another pre-validation check not shown in Fig. 1 enables to determine if rapid pacing is applied correctly based on the imaging data 2 during the cardiac surgical intervention such that the cardiac implant 3 can be moved through the native aortic valve annulus prior to deployment.

If all these results 5 are positive, the control unit 201 determines the position, orientation and shape of the cardiac implant 3 and of a cardiac anatomic feature 4 in the imaging data 2 shown in the display 6 on the right in Fig. 1.

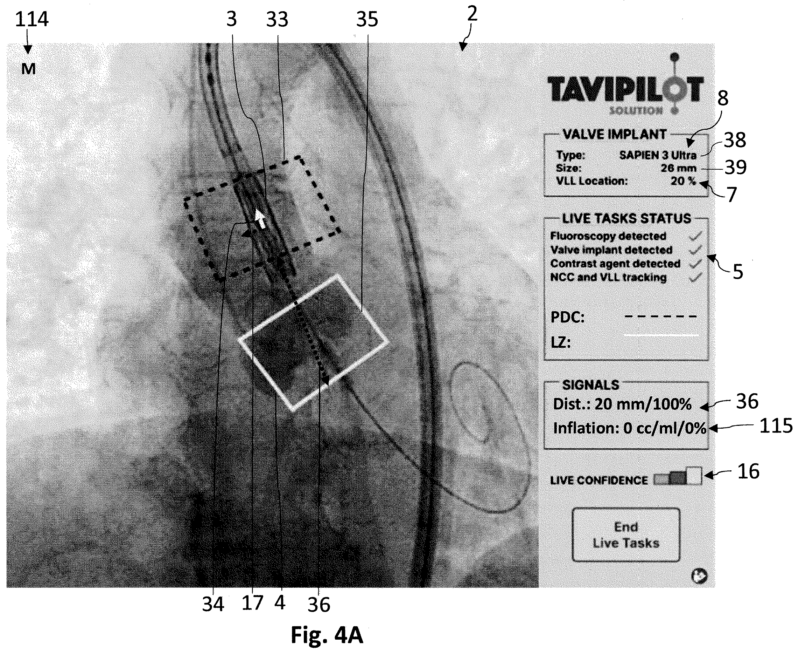

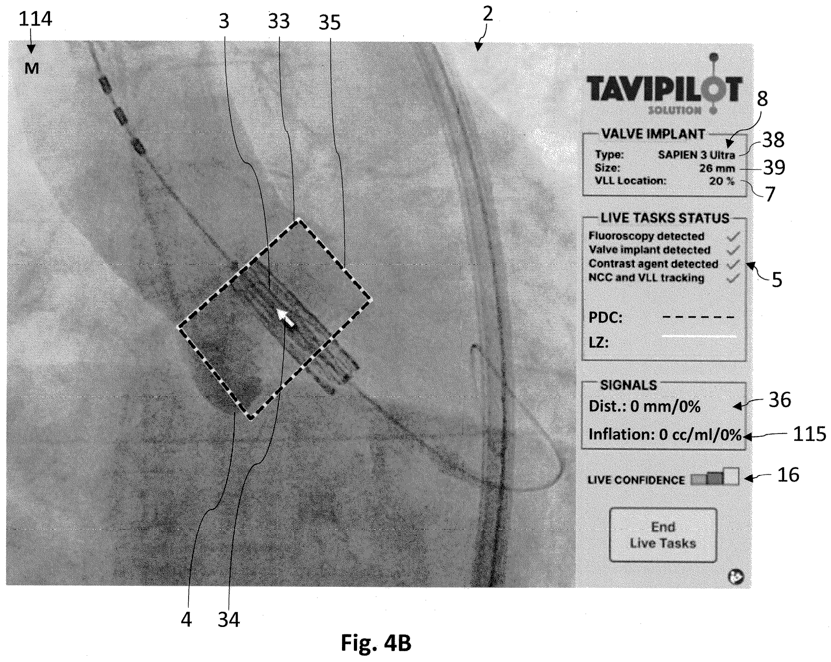

Further details and alternatives concerning the displayed visual outputs and visual data of the graphical user interface are provided in Figs. 2A - 2C and Figs. 4A and 4B.

The graphical user interface of Fig. 1 provides visual data to be overlaid with the fluoroscopy imaging data 2 to provide pertinent operational information to the clinician(s). The visual data in Fig. 1 on the right display 6 comprise a graphical representation of the geometric cardiac implant characteristic 32 which is determined based on the determined position and orientation of the cardiac implant 3. The geometric cardiac implant characteristic 32 is further determined and displayed based on the user input 7 indicative of the longitudinal location on the cardiac implant 3. The longitudinal location 31 of 20 % of a total cardiac implant length is exemplary included in Figs. 2A to 2C as double-headed white arrow originating on a distal end of the cardiac implant on the implant. The user input 7 indicative of the longitudinal location at 20% of a total length of the cardiac implant, the graphical representation of the geometric cardiac implant characteristic 32 will be included on the cardiac implant 3 at a position corresponding to this percentage of the current total length of the cardiac implant 3 with respect to the distal end of the cardiac implant 3 in the overlay. A width of this graphical representation of the geometric cardiac implant characteristic 32 perpendicular with respect to a longitudinal axis of the cardiac implant 3 in Fig. 1 corresponds to a fully expanded configuration of the cardiac implant 3. The graphical representation can further comprise a length corresponding to a length of the cardiac implant 3 in the fully expanded configuration (not shown in Fig. 1).

In addition, the graphical representation of the geometric cardiac implant characteristic 32 may comprise a length with respect to the longitudinal axis of the cardiac implant which corresponds to the length of the cardiac implant 3 in a fully expanded configuration (not shown in Fig. 1).

The results may be provided by the user interface by alternative binary visual indicators such as a green light for a positive result 5 and a red light for a negative result 5.

The fluoroscopy imaging data 2 displayed by the displays 6 in Fig. 1 further show a plurality of markers which indicate proximal edges and distal edges of the cardiac implant 3 which are only included for illustrative purposes. The control unit 201 may determine these edge markers. Based on these markers a current total length of the cardiac implant 3 can be reliably determined. Moreover, these markers may provide information on a non-uniform expansion pattern, i.e. if the distal edge markers denoting the distal end move to a greater degree with respect to the anatomy than the proximal edge markers.

The robotic controller 11 comprises a plurality of actuators 111 for actuating the medical instruments 112, a handle for the balloon catheter 113 on which the cardiac implant 3 is mounted, and a SAFARI guidewire 119. The robotic controller 11 in Fig. 1 is configured to advance and retract the SAFARI guidewire 119 to be placed in the left ventricle, advance and retract the balloon catheter 113, operate a SAPIEN 3 handle to tilt a distal tip of the balloon catheter 113, and operate a pressurizing unit for inflation of the balloon catheter 113. The robotic controller 11 is fixedly arranged on a robotic or a gravity-compensating manipulator arm 121 which is arranged on a solid support 122 or fixed to the bed of the patient.

Thereby, the robotic controller 11 can be used for navigation of the cardiac implant 3 to a target position such as the native aortic heart valve and deployment of the cardiac implant 3 via expansion of the balloon catheter 113. The robotic controller 11 may be configured for carrying out these steps of TAVI/TAVR by being remote controlled by a clinician or in a fully or partially autonomous manner. The robotic controller 11 may provide status data indicative of this manual, partially automated, or fully automated operational status to the control unit 201 and this operational status can be displayed in the user interface. A clinician can optionally select the desired operational status in the graphical user interface. The robotic controller 11 may be controlled in the fully or partially automated operational status via control commands generated and provided by the control unit 201.

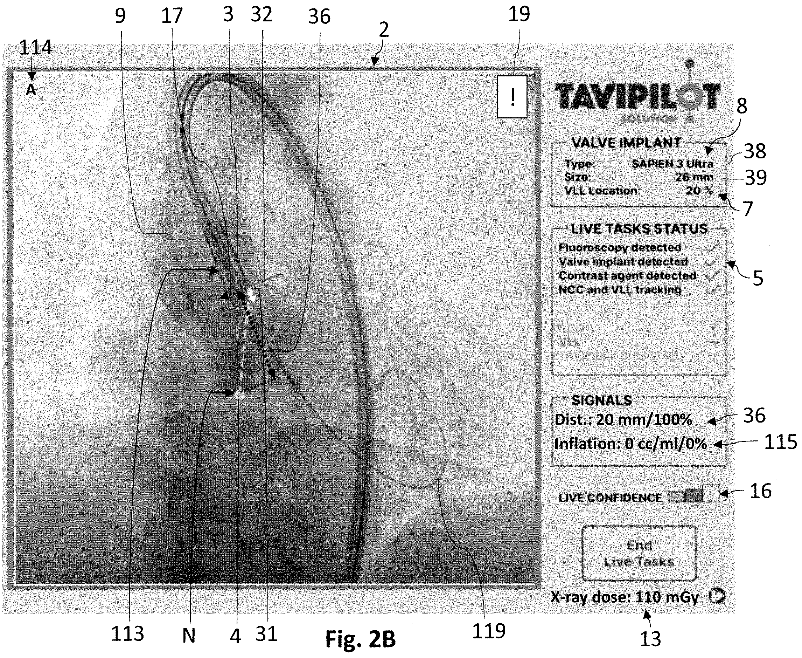

The status data 116 of the robotic controller 11 may be indicative of an actuation direction, e.g. advancement or retraction, of the medical instrument(s) 112 comprising or consisting of a sheath (not shown in Fig. 1), a balloon catheter 113, a guidewire, in particular a SAFARI guidewire 119, and/or a pigtail 9 (see Fig. 2B).

The status data 116 is indicative of slippage of rollers 117 with a medical instrument 112 or torque applied by the rollers 117. The rollers 117 are functionally connected to the actuator(s) 111 of the robotic controller 11 such that the medical instrument(s) 112 may be advanced/retracted through the vasculature.

The status data 116 can further provide an inflation volume used for inflation of the balloon catheter 113 for deploying the cardiac implant 3 by a pressurizing unit 118. The status data 116 further include a contrast agent administration volume which is administered in the surgical procedure.

The status data indicative of the slippage, actuation direction, torque, and/or the inflation status can enable to determine a more exact position, shape, and/or orientation of the cardiac implant 3. The control unit is configured for comparing this data to a position, shape, and/or orientation of the cardiac implant detected in the imaging data 3 in a closed feedback loop to determine the more exact position, shape, and/or orientation of the cardiac implant.

After successful placement of the cardiac implant 3 in the aortic valve by expanding it to the fully expanded configuration, additional contrast agent is administered, in particular by operating the robotic controller 11 automatically, to determine an extent of paravalvular leakage. The previously described control unit is configured to determine a paravalvular leakage metric based on the imaging data 2 subject to contrast agent administration. This paravalvular leakage metric is then displayed in the graphical user interface (not shown in Fig. 1).

Figure 2A shows an embodiment of a graphical user interface for performing the computer-implemented method according to the invention carrying out sequential pre-validation checks on imaging data 2. A feed of imaging data 2 is provided by the previously described control unit and imaging device and displayed in the graphical user interface in real-time. The user inputs 7, 8, including a selected type 38 and size 39 of a cardiac implant 3, and the pre-validation checks and corresponding results 5 are essentially the same as described in Fig. 1. The results 5 of the previously described pre-validation checks are provided in the section labelled "LIVE TASK STATUS" of the graphical user interface. A short descriptive text is provided for every pre-validation check in this section.

Figure 2A further shows that no overlay is provided on displayed fluoroscopy imaging data 2 because the cardiac implant 3, e.g. the aortic valve implant, the contrast agent in the cardiac region, and the feature tracking could not be reliably carried out based on the fluoroscopy imaging data 2 in Fig. 2A, at least not in a region of interest in proximity of a target site, e.g. the native aortic valve. The control unit and graphical user interface are configured to carry out the pre-validation checks automatically once the cardiac implant 3 is advanced in proximity of the target site, e.g. less than 2 cm with respect to a cardiac anatomic feature.

The graphical user interface further provides the clinician with a command window labelled "End Live Tasks" to end these pre-validation checks such that the cardiac surgical procedure can be carried out manually.

Figure 2B shows the graphical user interface according to Fig. 2A providing only positive results 5 of the pre-validation checks and displaying an overlay on the fluoroscopy imaging data 2 including a cardiac implant 3 being advanced through the ascending aorta. The fluoroscopy imaging data 2 displayed in Fig. 2B shows administered radiopaque contrast agent in the cardiac region such that the aortic root of the patient is clearly visible, and the pre-validation check for contrast detection provides a positive result 5. The pre-validation checks for tracking of a cardiac anatomic feature 4 in form of the non-coronary cusp N and a geometric cardiac implant characteristic 32 which is designated as virtual lucent line (VLL) in the graphical user interface also provides a positive result 5.

Another pre-validation check not displayed in Figs. 2A - 2C determines if the cardiac implant 3 or the previously described geometric cardiac implant characteristic 32 is arranged less than 2 cm from the position of the cardiac anatomic feature 4 formed by a tip of the non-coronary cusp N. If this is the case, as shown in Fig. 2B, the imaging data 2 are overlaid by a graphical representation of the geometric cardiac implant characteristic 32 of the cardiac implant 3 and the cardiac anatomic feature 4.

The imaging data 2 with the overlaid additional visual data comprising the cardiac anatomic feature and the graphical representation of the geometric cardiac implant characteristic 32 are provided in the graphical user interface in real-time.

The geometric cardiac implant characteristic 32 is computed by locating the position, orientation and shape in form of the current length of the cardiac implant 3 in the imaging data 2. The geometric cardiac implant characteristic 32 is then overlaid on the cardiac implant 3 in the imaging data 2 at a longitudinal location which is spaced apart based on a percentage of the current longitudinal length from a distal end of the cardiac implant along its longitudinal axis based on the user input 7.

The positive results of the pre-validation checks are indicated by a checkmark next to a descriptive text describing the pre-validation check in the graphical user interface.

The position cardiac anatomic feature 4 in Fig. 2B is displayed as a dot overlaid on the fluoroscopy imaging data and the graphical representation as a previously described perpendicular line to the cardiac implant's longitudinal axis. Figure 2B further shows that the fluoroscopy imaging data 2 are further overlaid with a connecting line of the graphical representation of the geometric cardiac implant characteristic 32 and the cardiac anatomic feature 4 as a light dashed line to simplify identifying a distance between these features.

The geometric cardiac implant characteristic 32 is instead displayed at a position on the cardiac implant 3 selected based on the previously described user input 7 indicative of a previously described longitudinal location 31 (see Figs. 1 and 2A), in particular a variable longitudinal location 31 which frequently adjusts to a location corresponding to a current total longitudinal length percentage of the cardiac implant 3 which was included in this figure as a double-headed white arrow. This longitudinal location 31 exemplary corresponds to 20 % of the total length of the cardiac implant 3 measured from the lower end of the cardiac implant 3 shown in Fig. 2B. This position may be determined based on determining the distance between the previously described proximal and distal edge markers. However, the user input 7 may be chosen by the clinician such that a therefrom resulting implantation depth can be achieved which corresponds to the native anatomy of the patient and a suitable indication.

The fluoroscopy image data 2 in Fig. 2B further show a plurality of medical instruments 112 comprising a bent guidewire, such as a SAFARI guidewire 119, which is placed in the left ventricle, a balloon catheter 113 on which the cardiac implant 3 is mounted, a delivery sheath, and a pigtail 9. The balloon catheter can be advanced over the bent guidewire such that the aortic valve implant can be advanced into the aortic heart valve and deployed by inflating the balloon catheter. The pigtail 9 was positioned in proximity of the non-coronary cusp and is configured for administration of contrast agent.

A pre-validation check can be carried out to determine that the pigtail 9 was successfully positioned in the non-coronary cusp N/or in proximity of the non-coronary cusp and an according visual output may be displayed by the graphical user interface (not shown in Fig. 2B).

The user interface further provides visual data generated by a control unit of the system indicative of a longitudinal projected distance 36 in mm and/or percent of the total length of the cardiac implant 3 to the cardiac anatomic feature 4. A double-headed black arrow indicative of the longitudinal projected distance 36 was overlaid on the fluoroscopy imaging data 2 in Fig. 2B for illustrative purposes. The longitudinal projected distance 36 is measured as the position of the cardiac anatomic feature 4 projected on a longitudinal axis of the cardiac implant 3 in the current position/orientation of the cardiac implant 3 shown in the imaging data 2. Thereby, an eccentric cardiac anatomic feature 4 such as the non-coronary cusp N which is typically clearly visible during contrast agent administration which is often directly administered in the non-coronary cusp, can be used for reliably determining a distance to the cardiac implant 3.

Moreover, the user interface provides visual data indicative of an inflation status 115, e.g. by displaying an inflation volume in customary units such as cubic centimetres (cc) / millilitres (ml) and/or percent with respect to complete inflation of a balloon catheter to deploy the cardiac implant 3.

The geometric cardiac implant characteristic 32 has a width perpendicular to the longitudinal axis which corresponds to a fully expanded configuration of the cardiac implant 3 to simplify arranging the cardiac implant concentrically within the native aortic heart valve. The control unit is configured to determine the width of the fully expanded configuration of the cardiac implant 3 based on the type 38 and size 39 provided by the other user input 8.

Figure 2B further shows an eccentricity 17 being displayed which is determined based on the geometric cardiac implant characteristic 32 with respect to another anatomic feature formed by the width of the aorta perpendicular to the longitudinal axis at a location of the geometric cardiac implant characteristic 32.

This may further facilitate aligning the cardiac implant 3 concentrically in the aortic heart valve prior to deployment.

Figure 2B also shows a confidence status 16 being displayed by the graphical user interface which is determined based on a prevalidation check. The confidence status 16 comprises three confidence levels which are displayed by the graphical user interface. This confidence status 16 provides the clinician with information on a reliability of the imaging data for correct placement of the cardiac implant determined by the control unit. Similarly to this confidence status 16, the graphical user interface can further display visual data of a contrast confidence status, e.g. also having three levels, which is indicative of an amount of contrast agent in the cardiac region of the patient.

Figure 2B further shows an amount 13 indicative of a total X-ray exposure throughout the cardiac surgical procedure being displayed by the graphical user interface which may e.g. be provided in milligray mGy. The graphical user interface can also be configured to display an amount indicative of the administered contrast agent, in particular based on status data from the robotic controller indicative of the amount or determined based on the fluoroscopy imaging data 2.

Figure 2B further shows that the control unit 201 generates a warning 19, exemplary displayed by an exclamation mark in Fig. 2B, which is displayed in the graphical user interface if the geometric cardiac implant characteristic 32 is spaced apart beyond a predetermined threshold distance parallel to the longitudinal projected distance 36 of more than 2 mm from the cardiac anatomic feature 4. This provides an additional safety measure which brings malpositioning of the cardiac implant 3 to the attention of the clinician.

The graphical user interface of Fig. 2B further shows that an operational status 114 is displayed, which may be indicative of an automatic operation "A", a partially automatic operation, or a manual operation of the previously described robotic controller 11 (see Fig. 1).

Prior to crossing the aortic annulus with the cardiac implant, another pre-validation check can be carried out to determine if rapid pacing with a rapid pacing catheter is successfully applied to the heart based on the fluoroscopy imaging data 2. The control unit may be specifically configured to determine if the heart rate is increased, typically above 180 beats per minute for TAVI/TAVR procedures, based on a feed of the fluoroscopy imaging data 2 which is typically provided at 3 - 30 Hz. Alternatively, rapid pacing control data indicative of operation of the rapid pacing catheter may be received by the control unit and included in the graphical user interface.

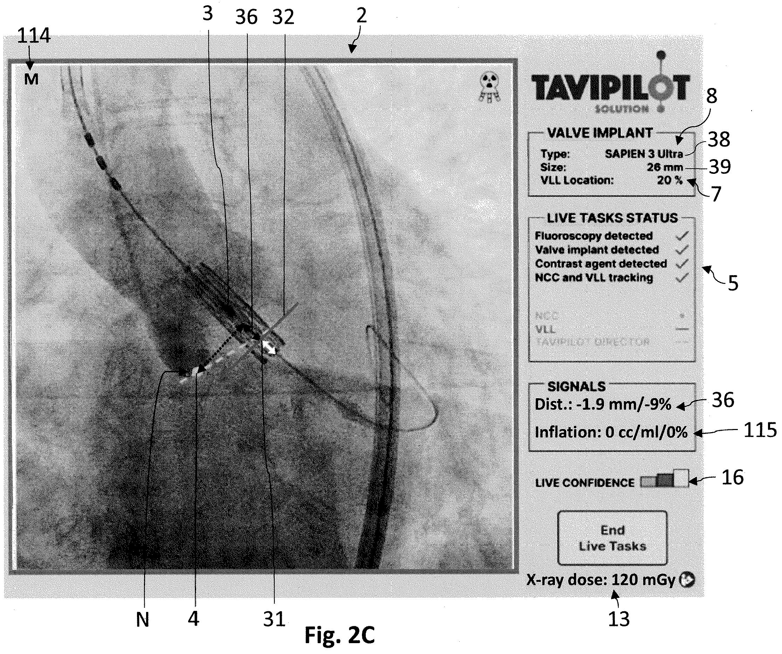

Figure 2C shows the graphical user interface according to the Figs. 2A and 2B displaying an overlay on the imaging data 2 including the cardiac implant 3 being advanced across the native aortic heart valve.

The pre-validation checks provide the results 5 as previously described as check marks and crosses. The fluoroscopy imaging data 2 are also overlaid with the geometric cardiac implant characteristic 32 indicative of a previously described longitudinal location on the cardiac implant 3 and a position of the cardiac anatomic feature 4 formed by a tip of the non-coronary cusp N.

Figure 2C shows that the cardiac implant 3 was advanced across the native aortic heart valve such that the previously described longitudinal projected distance 36 between the cardiac implant 3 and the cardiac anatomic feature 4 extends into the left ventricle with respect to the cardiac anatomic feature 4. By displaying an overlay on the fluoroscopy imaging data 2 in this manner, the cardiac implant 3 can be simply and reliably aligned by the clinician or a robotic controller 11 by aligning the connecting line between these the cardiac anatomic feature and the graphical representation such that it is parallel to the graphical representation of the geometric cardiac implant characteristic 32. Fig. 2C further shows that the longitudinal projected distance 36 is displayed to the clinician as mm or percent of the total length of the cardiac implant. The cardiac implant 3 SAPIEN 3 Ultra for the valve size 26 mm may for example have a total length of 20 mm in a non-deployed state.

The type 38, size 39, and the longitudinal location 31 determined by a user input 8 are also displayed in the graphical user interface of Fig. 2C. The longitudinal location 31 is a variable longitudinal location 31, i.e. the geometric cardiac implant characteristic 32 is displayed on the cardiac implant 3 at the longitudinal location 31 corresponding to 20 % of the current cardiac implant length in the imaging data 2. The distance corresponding to 20% of the current cardiac implant length is exemplary displayed as a double-headed white arrow in Fig. 2C.

The user interface further provides the user with the previously described inflation status 115 in cc/ml or percent of total inflation volume such that a clinician can monitor a deployment of the cardiac implant more precisely. The previously described confidence status 16 and X-ray dose amount 13 are also displayed in the user interface.

Status data indicative of the operational status 114 used for operating the previously described robotic controller 11 (see Fig. 1) is further displayed as manual mode "M" in the left upper edge of the graphical user interface.

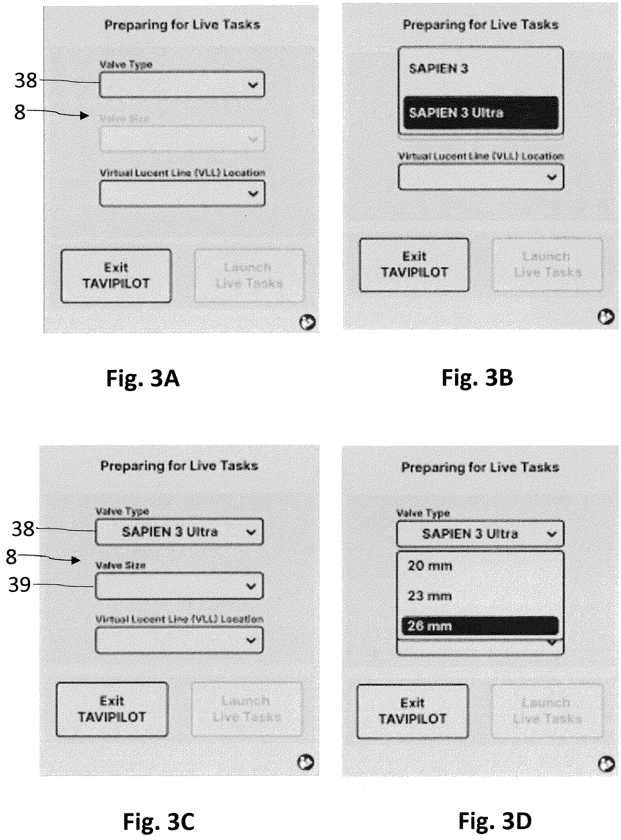

Figures 3A - 3G show an embodiment of a prompt of the graphical user interface for performing the computer-implemented method according to the invention for providing user inputs 7, 8 indicative of the type 38 and size 39 of the cardiac implant and indicative of a longitudinal location 31 (see Figs. 2B and 2C). The prompt includes a preparatory window prompting a user to enter user inputs 7, 8 prior to initialization of the pre-validation checks. After the user inputs were provided, the results and the fluoroscopy imaging data are displayed by the user interface side by side, optionally with the overlaid visual data.

Figures 3A and 3B show that a type 38 of a heart valve implant for implantation into the native aortic heart valve may be selected in a first step as a user input 8. In the example shown in Figs. 3A and 3B, the type 38 "SAPIEN 3 Ultra" is selected by a user.

Figures 3C and 3D show that a size 39 of this heart valve implant type 38 is selected by a user as a user input 8. In Figs. 3C and 3D, the user input "26 mm" is exemplary provided by a user.

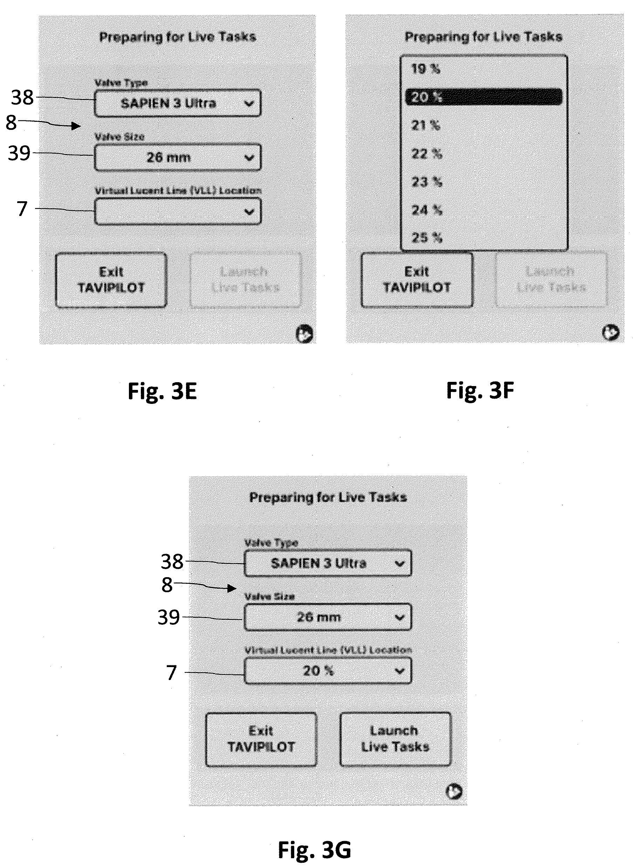

Figures 3E and 3F show that a user input 7 is entered which is indicative of the previously described longitudinal location 31 on the cardiac implant, e.g. with respect to a distal end of the cardiac implant. The user input 7 in Figs. 3E and 3F exemplary denotes 20 %, i.e. a longitudinal location on the cardiac implant being spaced apart 20 % of a current total length of the cardiac implant along a longitudinal axis of the cardiac implant in the imaging data from the distal end of the cardiac implant.

Figure 3G shows the entered user inputs 7, 8 and prompt the user to enter "Launch Live Tasks" such that the pre-validation checks can be carried out and the previously described graphical user interface can be provided. These user inputs 7, 8, in particular the user input indicative of the longitudinal location 31 which predetermines a target implantation depth may be amended throughout the surgical implantation procedure by re-opening the window "Preparing of Live Tasks".

Figures 4A and 4B show another embodiment of a graphical user interface for performing the computer-implemented method according to the invention including a predicted deployed configuration 33 and a landing zone 35. The predicted deployed configuration 33 is displayed as a dashed rectangular shape and the landing zone 35 is displayed as a white rectangular shape in Figs. 4A and 4B. The imaging data 2 and the overlay are provided by the graphical user interface in real-time.

The user inputs 7, 8 and the pre-validations checks are carried out similarly as previously described, i.e. providing the longitudinal location and the type and size of the cardiac implant. Figure 4A shows that results 5 of the pre-validation checks are positive indicated by checkmarks. The pre-validation checks determined (i) that fluoroscopy imaging data is detected, (ii) that a cardiac implant 3 is detected, and (iii) that contrast agent is detected in the aortic root, and (iv) that the non-coronary cusp and virtual lucent line based on the user input are reliably detected. A confidence status 16, an inflation status 115, and a mode of operation 114 is also provided in Figs 4A and 4B as previously described.

Instead of providing an overlay including a previously described graphical representation of a geometric cardiac implant characteristic which indicates the longitudinal location on the cardiac implant 3, the landing zone 35 is displayed directly in Figs. 4A and 4B. The landing zone 35 is determined based on a position of the detected cardiac anatomic feature 4 which is the tip of the non-coronary cusp as previously described in Figs. 2A to 2C. This landing zone 35 is further compensated based on the user input 7 indicative of the longitudinal location on the cardiac implant, e.g. by 20% with respect to the distal end of the cardiac implant 3. Thereby, the clinician can be provided with visual feedback where the cardiac implant 3 is to be placed based on this user input 7. The graphical user interface may enable re-entering a different user input 7 during the surgical procedure such that the landing zone 35 can be repositioned based on the clinician's expertise.

Alternatively, the user input may denote a displacement of the landing zone 35 with respect to the cardiac anatomic feature 4 directly.

The predicted deployed configuration 33 in Figs. 4A and 4B is determined based on the position and orientation of the cardiac implant 3 in the imaging data 2. The predicted deployed configuration 33 is further compensated for a longitudinal displacement 34 which is included in Figs. 4A and 4B as a white solid-line arrow for illustrative purposes. This longitudinal displacement 34 is mainly caused by a non-uniform expansion pattern of the cardiac implant 3 which can differ, depending on the type 38 and the size 39 of the cardiac implant 3 which may be considered in the compensation. This compensation may be switched on and off in the graphical user interface according to a preference of the clinician.

The rectangular shape of the predicted deployed configuration 33 and the landing zone 35 corresponds to a predicted width and length of the cardiac implant 3 in a fully expanded configuration. These shapes may be corrected based on the user input of the type 38 and the size 39 of the cardiac implant 3. Based on a large amount of training imaging data, a machine learning model may reliably predict these shapes and/or longitudinal displacement for different types 38 and sizes 39 of cardiac implants based on the provided imaging data 2.

Figures 4A and 4B further show that the predicted deployed configuration 33 is brought into alignment with the landing zone 35 such that the cardiac implant 3 can be safely and reliably deployed in the aortic heart valve at the desired implantation depth. This can be achieved by manual and/or fully or partially automatic actuation of the previously described robotic controller. This predicted deployed configuration 33 and the landing zone 35 which are overlaid on the fluoroscopy image 2 may assist the clinician performing a navigation and deployment or supervising a partially or fully automated navigation and deployment. The predicted deployed configuration 33 and the landing zone 35 are indicative of longitudinal and lateral positioning of the cardiac implant 3 with respect to the cardiac anatomic feature(s). Hence, a longitudinal and lateral positioning of the cardiac implant 3 can be facilitated.

The graphical user interface further displays visual data indicative of a longitudinal projected distance 36 between the predicted deployed configuration 33 and the landing zone 35 in millimetre or percent of a total length of the cardiac implant 3. This longitudinal projected distance 36 is further displayed as an overlay of a double-headed dashed black arrow originating at a lower edge of the predicted deployed configuration 33 extending along a longitudinal axis of the cardiac implant 3 leading to a lower edge of the landing zone 35.

In addition, an eccentricity 17 may be determined based on the position of the position of the cardiac implant 3 and another cardiac anatomic feature formed by a local width of the body duct outline, e.g. the aortic root (not shown in Figs. 4A and 4B). This eccentricity 17 may be displayed as an overlay on the fluoroscopy imaging data 2 in the graphical user interface and/or as a value next to the imaging data 2 similarly to the longitudinal projected distance 36. This may facilitate aligning the cardiac implant 3 in a concentric manner with respect to the body duct outline,e.g. via a tilt adjustment operated by the previously described handle.

Figure 5A shows a schematic illustration of a body duct outline 12 overlaid onto displayed fluoroscopy imaging data 2 acquired in a three-cusp view. A cardiac implant 3 is arranged on a balloon catheter 113 which is navigated to the aortic heart valve of the patient to be deployed by using a previously described plurality of medical instrument 112 and robotic controller 11 (see Fig. 1).

The body duct outline 12 in Fig. 5A is exemplary determined based on fluoroscopy imaging data 2 which were acquired during contrast agent administration to the cardiac region of the patient. Thereby, the body duct outline 12 including a contour of the aortic root region comprising the cusps N, R, L can be computed and overlaid on the imaging data 2. This body duct outline 12 may be continuously overlaid on the imaging data 2, even after dissipation of the contrast agent, to facilitate exact navigation of the cardiac implant 3.

In the three-cusp view, the angles of the fluoroscopy imaging device 21 (see Fig. 1) are adjusted such that the non-coronary cusp N, the right coronary cusp R, and the left coronary cusp L are equidistantly spaced apart in the displayed fluoroscopy imaging data 2 (see also Fig. 5B). The fluoroscopy imaging device 21 used for acquiring the fluoroscopy imaging data 2 is a C-arm and the angles to be adjusted may be a cranial-caudal (CR/CA) tilt angle and a left anterior oblique/right anterior oblique (LAO/RAO) rotational angle. The graphical user interface may display a visual indicator 41 if the C-arm is in the three-cusp orientation denoted by "3C" or in the two-cusp orientation denoted by "2C". Even though not explicitly shown in Fig. 5A, the user interface also provides the previously described results 5, user inputs 7, 8 and visual data to be overlaid on the imaging data 2.

The user interface can further provide control instructions which are generated based on the received imaging data 2 to align the imaging device 21 (see Fig. 1) in the three-cusp or two-cusp orientation, e.g. by adjusting the above-mentioned angles. Alternatively, a control command to achieve these control instructions generated by the previously described control unit can be directly transmitted to the imaging device 21 based on a user input to achieve the two- or three-cusp view.

Figures 5B and 5C show a schematic illustration of the three-cusp and two-cusp view respectively. In the three-cusp view shown in Fig. 5B, the cusps N, R, L are arranged equidistantly, such that the heart valve implant 3 can be arranged centrically and overlappingly with the right coronary cusp R. In the two-cusp view shown in Fig. 5C, two of the cusps, typically the left and right coronary cusps, are arranged behind each other such that they appear to be co-planar in the imaging data. Changing between these views may enable clinicians to verify if the heart valve implant 3 is concentrically arranged in the aortic heart valve.

Figure 5A further shows a that the trajectory of the cardiac implant 3 was continuously detected in the fluoroscopy imaging data 2. The detected trajectories are processed by the control unit to display a past trajectory 181 of the cardiac implant 3 as an overlay on the currently received imaging data 2 in real-time. The past trajectory 181 is displayed as a narrow dashed white line in Fig. 5A which terminates at a center of the cardiac implant 3. Figure 5A further shows a predicted future trajectory 182 being displayed as a long-dashed white line originating from the center of the cardiac implant 3 which is overlaid onto the imaging data 2. The future trajectory 182 is generated based on the position of the anatomic feature 4, i.e. the tip of the non-coronary cusp N, the body duct outline 12, and the position of the cardiac implant 3. The future trajectory 182 may be predicted such that (i) the cardiac anatomic feature 4 can be aligned with the previously described geometric cardiac implant characteristic or (ii) the previously described predicted deployed configuration 33 can be aligned with the landing zone 25 (see Figs. 4A and 4B).

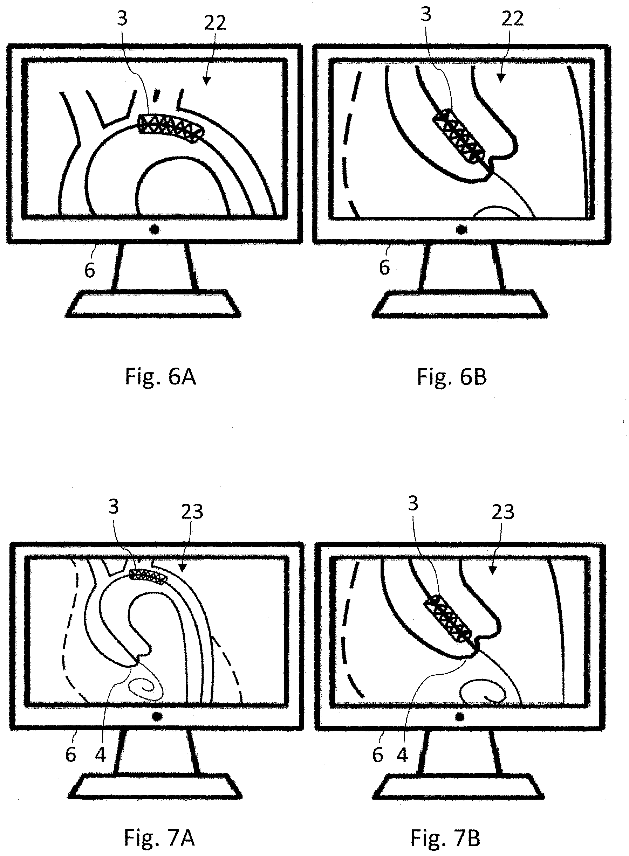

Figures 6A and 6B show a schematic illustration of centered imaging data 22 being displayed on a display 6. After receiving the imaging data 2 from the imaging device 21 (see Fig. 1), the imaging data 2 are processed by a control unit such that a cardiac implant 3 is arranged in a center region if the centered imaging data 22 are displayed on the display 6 in the graphical user interface. The graphical user interface and/or control unit processing the imaging data 2 may be a previously described graphical user interface and/or control unit. The graphical user interface may further provide for a user input which enables switching between displaying the centered imaging data 22 and non-centered imaging data 2. Centering the cardiac implant 3 being displayed may facilitate navigation and/or deployment of the cardiac implant 3, in particular during inflation of a balloon catheter.

Figure 7A and 7B show a schematic illustration of imaging data 23 which were pre-processed to adjust a zoom level based on a distance between a cardiac implant 3 and a cardiac anatomic feature 4. The cardiac anatomic feature 4 may be a tip of the non-coronary cusp as previously described. Figure 7A shows that the imaging data 23 (see e.g. Fig. 1) were pre-processed prior to be displayed to have a rather low zoom level based on the cardiac implant 3 and the cardiac anatomic feature 4 being spaced apart by a large distance. Figure 7B shows that the pre-processes imaging data 23 were processed prior to being displayed to have a rather high zoom level because the cardiac implant 3 and the cardiac anatomic feature 4 are arranged a small distance apart from each other. The zoom levels are chosen such that both the cardiac implant 3 and the cardiac anatomic feature 4 are always included in the pre-processed imaging data 23 irrespective of the distance between them. Adjusting the zoom level in this manner may provide the clinician or a control unit configured for partial or fully automated control of the medical instruments with less non-relevant data during the critical phase of deploying the cardiac implant 3 in proximity of the cardiac anatomic feature 4.

A previously described graphical user interface and/or control unit may be configured to enable switching between generating and displaying pre-processed imaging data 23 and displaying imaging data 2 which were not adjusted based on the zoom level.

Both the centered imaging data 22 and/or the pre-processed imaging data may be generated and displayed in the previously described graphical user interfaces (see Figs. 2A - 2C and Figs. 4A and 4B).

## Claims

1. A computer-implemented method for assisting in a cardiac surgical procedure, the method comprising:
- receiving a plurality of imaging data (2) from an imaging device (21), in particular fluoroscopy imaging data from a fluoroscopy imaging device,
- optionally verifying in a first pre-validation check if the imaging data (2) has a correct format, dimension, metadata attribute, and/or size,
- carrying out, in particular if the imaging data (2) was verified, at least one of the following pre-validation checks:
∘ automatically determining if a cardiac implant (3), in particular a heart valve implant, is included in the imaging data (2),
∘ automatically determining if a contrast agent in the cardiac region is detected in the imaging data (2),
∘ automatically determining if a cardiac anatomic feature (4), in particular the non-coronary cusp (N), is included in the imaging data (2) or can be determined based on the imaging data (2), and
- Providing an output indicative of at least one result (5) of the at least one pre-validation check, in particular of every pre-validation check, preferably a visual output to be displayed on a display (6).

2. The computer-implemented method according to claim 1, wherein if the cardiac implant (3) and/or the cardiac anatomic feature (4) is included in the imaging data (2) or can be determined based on the imaging data (2), the pre-validation check further includes:
- verifying if the imaging data (2) is sufficient to determine a position, a shape, and/or an orientation of the cardiac implant (3) and/or the cardiac anatomic feature (4), in particular with respect to each other.

3. The computer-implemented method according to one of the preceding claims, wherein the pre-validation check comprises, in particular if the imaging data are verified to be sufficient,
- determining the position, the shape, and/or the orientation of the cardiac implant (3) and/or the cardiac anatomic feature (4) and
- providing visual data comprising the position, the shape, and/or the orientation of the cardiac implant (3) and/or the cardiac anatomic feature (4), in particular with respect to each other.

4. Computer-implemented method according to claim 3, wherein the pre-validation check comprises:
- receiving a user input (7) indicative of a longitudinal location (31) on the cardiac implant (3), in particular in the form of a percentage of a total longitudinal length of the cardiac implant (3),
- optionally receiving another user input (8) indicative of a type (38) and/or a size (39) of the cardiac implant (3),
- processing the position, the shape, and/or the orientation of the cardiac implant (3) to determine a geometric cardiac implant characteristic (32) which is indicative of the longitudinal location (31), and
- generating and providing visual data including data for displaying the geometric cardiac implant characteristic (32) in the imaging data (2) as a graphical representation, and wherein
the graphical representation preferably has (i) a width perpendicular to a longitudinal axis of the cardiac implant (3) at a reference point of the cardiac implant (3) in the imaging data (2) corresponding to the longitudinal location (31) of the user input (7), and optionally also (ii) a length of the cardiac implant (3) parallel to the longitudinal axis of the cardiac implant (3),
- wherein the width, and in particular the length, optionally correspond to the cardiac implant (3) in a fully expanded configuration and are determined based on the other user input (8) of the type (38) and/or the size (39) of the cardiac implant (3).

5. Computer-implemented method according to one of the claims 3 or 4, wherein the pre-validation check comprises:
- optionally receiving a user input (8) indicative of a type (38) and/or size (39) of the cardiac implant (3),
- generating a predicted deployed configuration (33) of the cardiac implant (3) in the fully expanded configuration based on the position, the shape and/or the orientation of the cardiac implant (3) with respect to the imaging data (2), wherein
the predicted deployed configuration (33) optionally comprises or consists of a computed visualization of the cardiac implant (3) in the fully expanded configuration,
- optionally compensating a position of the predicted deployed configuration (33) of the cardiac implant (3) for a longitudinal displacement (34) with respect to the current position of the cardiac implant (3) in the imaging data (2) caused by a non-uniform expansion pattern of the cardiac implant (3), in particular based on the type (38) and/or size (39) of the cardiac implant (3),
- providing visual data comprising the predicted deployed configuration, in particular compensated for the non-uniform expansion pattern.

6. Computer-implemented method according to one of the claims 3 to 5, wherein the pre-validation check comprises:
- generating a landing zone (35) of the cardiac implant (3) in a fully expanded configuration based on the position, the shape, and/or the orientation of the cardiac anatomic feature (4) with respect to the imaging data (2), wherein the landing zone (35) in particular comprises or consists of a computed visualization of the cardiac implant (3),
- optionally receiving a user input (7) indicative of a longitudinal location (31) on the cardiac implant (3), in particular in the form of a percentage of a total longitudinal length of the cardiac implant (3),
- optionally compensating the landing zone (35) of the cardiac implant (3) based on the longitudinal location (31) on the cardiac implant (3),
- providing visual data comprising the landing zone (35).

7. The computer-implemented method according to one of the claims 3 to 6, wherein the method comprises:
- determining a distance between (i) the position of the cardiac implant (3) or the geometric cardiac implant characteristic (32) according to claim 4 and (ii) the position of the cardiac anatomic feature (4) in a projection on the longitudinal axis,
- providing visual data comprising the longitudinal projected distance (36).

8. The computer-implemented method according to one of the claims 3 to 7, wherein the method comprises:
- Determining an eccentricity (17) between the position of the cardiac implant (3) or the geometric cardiac implant characteristic (32) according to claim 4 and the position of the cardiac anatomic feature (4),
- Providing visual data comprising the eccentricity (17).

9. The computer-implemented method according to one of the preceding claims, wherein the pre-validation check comprises determining if a pigtail (9) is included in the imaging data (2), and if so,
in particular determine if the pigtail (9) is positioned within one of the cusps (L, R, N), preferably determine if the pigtail (9) is positioned within the non-coronary cusp (N), the right coronary cusp (R), or the left coronary cusp (L) .

10. The computer-implemented method according to one of the preceding claims, wherein the pre-validation check comprises processing of the imaging data (2) to determine if the cardiac implant (3) or the geometric cardiac implant characteristic (32) is arranged in proximity of (i) the cardiac anatomic feature (4) and/or (ii) the pigtail (9) according to claim 9, and optionally if the cardiac implant (3) or the geometric cardiac implant characteristic (32) is spaced apart less than 5 cm, in particular less than 3.5 cm, preferably less than 2 cm, from the cardiac anatomic feature (4) and/or the pigtail (9).

11. The computer-implemented method according to one of the preceding claims, wherein the pre-validation check comprises processing the imaging data (2) to determine if the imaging data (2) were acquired in a two-cusp view, in a three-cusp view, or in another view, and wherein
the method optionally comprises generating at least one control instruction to move the imaging device (21) to achieve the two-cusp view or the three-cusp view, and
wherein:
- (i) the output comprises the at least one control instruction and/or
- (ii) at least one control command for the imaging device (21) is generated and transmitted to the imaging device (21) to achieve the at least one control instruction, and
wherein
the method in optionally comprises providing visual data to be displayed on a display (6) indicative of whether the imaging data (2) were acquired in the two-cusp view, in the three-cusp view, or in another view.

12. The computer-implemented method according to one of the preceding claims, wherein the pre-validation check comprises receiving or retrieving status data (116) from at least one robotic controller (11), wherein the status data may (116) be indicative of at least one of:
- (i) an actuation direction of the at least one medical instrument (112) driven by at least one actuator (111) of the robotic controller (11),
- (ii) sensor data indicative of slippage of the at least one actuator (111) driving the at least one medical instrument (112),
- (iii) a status of the robotic controller (11), in particular an operational status (114) indicating if the robotic controller (11) is operated manually, partially automatically, or fully automatically,
- (iv) an inflation status (115) of a balloon catheter (113) for deploying the cardiac implant (3), in particular including a volume of inflation fluid used for inflation,
- (v) a contrast agent status, in particular including a volume of contrast agent administered, and
- (vi) a torque of the at least one actuator (111) for driving the at least one medical instrument (112), and wherein
the status data (116) is in particular processed for determining a predicted position, shape, and/or orientation of the cardiac implant (3) or the geometric cardiac implant characteristic (32) based on the status data (116) and providing visual data comprising the predicted position, shape, and/or orientation.

13. The computer-implemented method according to one of the preceding claims, wherein the method comprises determining a body duct outline (12), in particular an aorta outline, based on the imaging data (2), optionally using a pre-trained machine learning model,
wherein the body duct outline (12) is determined based on at least one of:
- (i) prior and/or current imaging data (2) acquired during administration of contrast agent,
- (ii) prior and/or current imaging data (2) acquired without administration of contrast agent, and
- (iii) pre-operational imaging data, preferably computer tomography imaging data, and
providing visual data comprising the body duct outline (12) to be overlaid on the imaging data (2).

14. The computer-implemented method according to one of the preceding claims, wherein the pre-validation check may comprise determining if rapid pacing is successfully applied to the patient, in particular based on (i) the imaging data (2), (ii) heartbeat sensor data, and/or (iii) received rapid pacing control data for operating a rapid pacing device.

15. The computer implemented method according to one of the preceding claims, wherein the method comprises providing the imaging data (2) to be displayed on a display (6), in particular together with the output, and wherein
the imaging data (2) are preferably overlaid with at least one of:
- the at least one position, shape, and/or orientation of the cardiac implant (3) and/or the cardiac anatomic feature (4) according to claim 3,
- the geometric cardiac implant characteristic (32) according to claim 4,
- the predicted deployed configuration (33) according to claim 5,
- the generated landing zone (35) according to claim 6,
- a visual indicator indicative of the longitudinal projected distance (36) according to claim 7 or the eccentricity (17) according to claim 8,
- the predicted position, shape, and/or orientation of the cardiac implant (3) and/or the geometric cardiac implant characteristic according to claim 12, and
- the body duct outline (12) according to claim 13.

16. The computer-implemented method according to claim 15, wherein the method comprises at least one of:
- (i) generating visual data comprising a warning (19),
- (ii) generating a control command for preventing actuation of the robotic controller (11) and transmitting the control command to the robotic controller (11),
if the predicted position, shape, and/or orientation of the cardiac implant (3) or the geometric cardiac implant characteristic (32) is spaced apart beyond a predetermined threshold distance relative to the cardiac anatomic feature (4), wherein the predetermined threshold distance in particular is less than 3 mm, preferably less than 2 mm.

17. The computer-implemented method according to one of the preceding claims, wherein the method comprises:
- determining a confidence status (16) based on the at least one result (5) of the at least one pre-validation check to classify a suitability of the imaging data (2) or the at least one result (5) for performing a placement of the cardiac implant (3) in a heart valve, in particular the heart valve implant in the aortic heart valve,
- providing in particular at least three confidence levels (16) indicating at least three different suitability regimes, and
- providing visual data indicative of the confidence levels (16), preferably by providing visual data for displaying a bar diagram and/or a colour scheme, in particular a traffic light colour scheme.

18. The computer-implemented method according to one of the preceding claims, wherein the method comprises:
- determining a contrast confidence status indicative of an amount of contrast agent in the cardiac region in the imaging data,
- providing in particular at least three contrast confidence levels indicating at least three different regimes of contrast agent amounts, and
- providing visual data indicative of the contrast confidence levels, preferably by providing visual data for displaying a bar diagram and/or a colour scheme, in particular a traffic light colour scheme.

19. The computer-implemented method according to one of the preceding claims, wherein the method comprises determining an amount (13) indicative of (i) a total X-ray exposure and/or (ii) contrast agent administration based on the imaging data (2) and providing visual data comprising this amount (13) to be displayed on the display (6).

20. The computer-implemented method according to one of the preceding claims, wherein the method comprises processing the imaging data (2) such that the cardiac implant (3) included in the imaging data (2) is displayed in a center of a displayed image based on the imaging data (2) and providing the centered imaging data (22) to be displayed on the display (6).

21. The computer-implemented method optionally according to one of the claims 3 to 20, wherein the method comprises:
- determining a distance between a current position of the cardiac implant (3) and a current position of the cardiac anatomic feature (4),
- pre-processing the imaging data (2) to be displayed by adjusting a zoom level before providing it such that the zoom level is higher when the distance is smaller and lower when the distance is larger, and wherein
the zoom level is preferably chosen such that the cardiac implant (3) and the cardiac anatomic feature (4) are included or can be determined based on the pre-processed imaging data (23) irrespective of the zoom level.

22. The computer-implemented method according to one of the preceding claims, wherein the method comprises at least one of:
- continuously detecting a trajectory of the position of the cardiac implant (3) with respect to the imaging data (2) and processing the detected trajectories to determine a past trajectory (181),
- predicting a future trajectory (182) of the cardiac implant (3) with respect to the imaging data (2) based on the position of the cardiac anatomic feature (4), and in particular the body duct outline (12),
and providing visual data for displaying the past and/or future trajectory.

23. The computer-implemented method according to one of the preceding claims, wherein the method comprises:
- determining a paravalvular leakage metric indicative of paravalvular leakage of a fully deployed cardiac implant (3) after the cardiac implant (3) was converted to the fully expanded configuration based on the imaging data (2), in particular imaging data acquired under the influence of contrast agent, and
- providing visual data for displaying the paravalvular leakage metric.

24. The computer-implemented method according to one of the preceding claims, wherein the method comprises sequentially providing display data indicative of which pre-validation check is currently carried out, and in particular indicative of which pre-validation check was carried out already, preferably together with the corresponding output indicative of the result of the corresponding pre-validation check.

25. The computer-implemented method according to one of the preceding claims, wherein the method comprises:
- determining (i) the inflation status of the balloon catheter (113) for deploying the cardiac implant (3), in particular including the volume of inflation fluid used for inflation, and/or
(ii) the contrast agent status, in particular including a volume of contrast agent administered based on the imaging data (2), and
- providing visual data to be displayed on the display (6) indicative of the inflation status and/or the contrast agent status.

26. A system (101) configured for carrying out the computer-implemented method according to one of the preceding claims, wherein the system comprises a control unit (201) for receiving the imaging data (2) and providing the output, and in particular the visual data,
in particular a display for displaying the output, and in particular the visual data, and
preferably a robotic controller (11) according to claim 12 connected to the control unit (201).

27. A computer program product which, when the program is executed by a computer, causes the computer to carry out the steps of the computer-implemented method of one of the claims 1 - 25.

28. A computer-readable storage medium comprising instructions which, when executed by a computer, cause the computer to carry out the steps of the computer-implemented method of one of the claims 1 - 25.

29. A computer-implemented method for providing visual information to a user for assistance in a cardiac surgical procedure, the method comprising:
- receiving a user input (7) indicative of a longitudinal location (31) on a cardiac implant (3), in particular a percentage of a total longitudinal length of the cardiac implant (3),
- optionally receiving another user input (8) indicative of a type (38) and/or a size (39) of the cardiac implant (3),
- receiving a plurality of imaging data (2) from an imaging device (21), in particular a fluoroscopy imaging device,
- determining a position, shape, and/or orientation of the cardiac implant (3) based on the imaging data (2),
- processing the position, shape, and/or orientation of the cardiac implant (3) based on the user input to determine a geometric cardiac implant characteristic which is indicative of the longitudinal location,
- generating and providing visual data including data for displaying the geometric cardiac implant characteristic in the imaging data (2) as a graphical representation, and wherein
the graphical representation preferably has (i) a width perpendicular to a longitudinal axis of the cardiac implant (3) at a reference point of the displayed cardiac implant (3) corresponding to the longitudinal location (31) of the user input (7), and optionally also (ii) a length of the cardiac implant (3) parallel to the longitudinal axis of the cardiac implant (3),
- wherein the width, and in particular the length, optionally correspond to the cardiac implant (3) in a fully expanded configuration and are determined based on the other user input (8) indicative of a type (38) and/or a size (39) of the cardiac implant (3).

30. Computer-implemented method according to claim 29, wherein the method comprises providing the imaging data (2) to be displayed on a display, in particular together with the visual data, and preferably with the other user input (8) indicative of the type (38) and/or the size (39) of the cardiac implant (3), and wherein the imaging data (2) are preferably overlaid with the data for displaying the geometric cardiac implant characteristic.

31. Computer-implemented method according to one of the claims 29 or 30, wherein the method comprises:
- Receiving a user input indicative of (i) a volume of inflation fluid to be used for inflation of a balloon catheter and/or (ii) a volume of contrast agent, in particular a maximum volume, to be administered during the cardiac surgical procedure,
- Optionally receiving status data indicative of (i) the volume of inflation fluid used for inflation of a balloon catheter and/or (ii) the volume of contrast agent administered during the cardiac surgical procedure,
- Providing visual data indicative of the volume, in particular corrected for the volume used and/or administered, to be displayed on a display (6).

32. A computer program product which, when the program is executed by a computer, causes the computer to carry out the steps of the computer-implemented method of one of the claims 29 - 31.

33. A computer-readable storage medium comprising instructions which, when executed by a computer, cause the computer to carry out the steps of the computer-implemented method of one of the claims 29 - 31.
